(19) Europäisches Patentamt / European Patent Office / Office européen des brevets

(11) EP 1 348 760 B1

(12) EUROPÄISCHE PATENTSCHRIFT

(45) Veröffentlichungstag und Bekanntmachung des Hinweises auf die Patenterteilung:
**13.01.2010 Patentblatt 2010/02**

(51) Int Cl.:
***C12N 9/16*** *(2006.01)* ***C12N 5/10*** *(2006.01)*

(21) Anmeldenummer: **03006426.5**

(22) Anmeldetag: **21.03.2003**

# (54) Erzeugung von schwach aktiven oder inaktiven Mutanten von Alkalischer Phosphatase und deren Expression in Hefe

Production of inactive mutants or mutants with a low activity of an alkaline phosphatase and their expression in yeast

Production de mutants d'une phosphatase alcaline inactifs ou ayant une activité reduite et leur expression dans la levure

(84) Benannte Vertragsstaaten:
**AT BE BG CH CY CZ DE DK EE ES FI FR GB GR HU IE IT LI LU MC NL PT RO SE SI SK TR**

(30) Priorität: **25.03.2002 DE 10213201**

(43) Veröffentlichungstag der Anmeldung:
**01.10.2003 Patentblatt 2003/40**

(73) Patentinhaber:
- **Roche Diagnostics GmbH**
  **68305 Mannheim (DE)**
  Benannte Vertragsstaaten:
  **DE**
- **F.HOFFMANN-LA ROCHE AG**
  **4070 Basel (CH)**
  Benannte Vertragsstaaten:
  **AT BE BG CH CY CZ DK EE ES FI FR GB GR HU IE IT LI LU MC NL PT SE SI SK TR**

(72) Erfinder:
- **Mueller, Rainer, Dr.**
  **82377 Penzberg (DE)**
- **Thalhofer, Johann-Peter, Dr.**
  **82362 Weilheim (DE)**
- **Geipel, Frank, Dr.**
  **82377 Penzberg (DE)**
- **Hoelke, Werner, Dr.**
  **82377 Penzberg (DE)**
- **Kirschbaum, Thomas, Dr.**
  **81543 Muenchen (DE)**

(74) Vertreter: **Jung, Michael**
**Roche Diagnostics GmbH**
**Patentabteilung**
**68298 Mannheim (DE)**

(56) Entgegenhaltungen:
**EP-A- 0 955 369 EP-A- 1 176 205**
**US-A- 5 707 853**

- **WEISSIG H ET AL: "CLONING AND EXPRESSION OF THE BOVINE INTESTINAL ALKALINE PHOSPHATASE GENE: BIOCHEMICAL CHARACTERIZATION OF THE RECOMBINANT ENZYME" BIOCHEMICAL JOURNAL, PORTLAND PRESS, LONDON, GB, Bd. 290, Nr. 2, 1. März 1993 (1993-03-01), Seiten 503-508, XP002070998 ISSN: 0264-6021 -& DATABASE EMBL [Online] EBI; 1. November 1990 (1990-11-01) WEISSIG H ET AL: "Alkaline phosphatase, intestinal precursor (EC 3.1.3.1)" retrieved from EMBL Database accession no. P19111 XP002259394**
- **MANES T ET AL: "GENETIC COMPLEXITY, STRUCTURE, AND CHARACTERIZATION OF HIGHLY ACTIVE BOVINE INTESTINAL ALKALINE PHOSPHATASES" JOURNAL OF BIOLOGICAL CHEMISTRY, AMERICAN SOCIETY OF BIOLOGICAL CHEMISTS, BALTIMORE, MD, US, Bd. 273, Nr. 36, 4. September 1998 (1998-09-04), Seiten 23353-23360, XP001029065 ISSN: 0021-9258**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **BESMAN M ET AL: "ISOZYMES OF BOVINE INTESTINAL ALKALINE PHOSPHATASE" JOURNAL OF BIOLOGICAL CHEMISTRY. (MICROFILMS), AMERICAN SOCIETY OF BIOLOGICAL CHEMISTS, BALTIMORE, MD, US, Bd. 260, Nr. 20, 15. September 1985 (1985-09-15), Seiten 11190-11193, XP000606610**

**Beschreibung**

Die Erfindung betrifft ein Verfahren zur rekombinanten Herstellung bzw. Expression von Mutanten einer eukaryontischen Alkalischen Phosphatase, die schwach aktiv oder inaktiv sind. Die Erfindung betrifft darüber hinaus codonoptimierte DNAs, basierend auf der Nukleinsäuresequenz, die für eine hochaktive Alkalische Phosphatase kodiert und die durch gezielte Mutagenese derart verändert wurde, dass sie für eine nur schwach aktive oder inaktive Alkalische Phosphatase kodiert. Die Erfindung betrifft ferner ein Verfahren zur Insertion der mutierten DNA in einen Vektor zur Expression in Hefe-Zellen sowie ein Verfahren zur Expression der Alkalischen Phosphatase-Mutanten in Hefe.

Alkalische Phosphatasen (AP) sind dimere, zinkhaltige, nichtspezifische Phosphomonoesterasen, die sowohl in prokaryontischen wie auch eukaryontischen Organismen, z.B. in E. coli und Säugern vorkommen (McComb et al., 1979 Alkaline Phosphatases Plenum Press, New York). Der Vergleich der Primärstruktur verschiedener Alkalischer Phosphatasen ergab einen hohen Homologiegrad (25-30% Homologie zwischen E. coli- und Säuger-AP; Millàn, 1988 Anticancer Res. 8, 995-1004; Harris, 1989 Clin. Chim. Acta 186, 133-150).

Im Menschen und höheren Tieren besteht die AP-Familie aus vier Mitgliedern, die in verschiedenen Genloci codiert sind (Millan, 1988 Anticancer Res. 8, 995-1004; Harris 1989 Clin. Chim. Acta 186, 133-150). Zur Familie der Alkalischen Phosphatasen zählen die gewebespezifischen APs (Placenta-AP (PLAP), Germzellen-AP (GCAP) und Darm-AP (IAP)) und die nicht-gewebespezifischen APs (TnAP), die vorwiegend in Leber, Niere und Knochen lokalisiert sind.

Eine entscheidende Eigenschaft der bislang bekannten APs ist die große Variabilität in der katalytischen Aktivität der Säuger-APs, die einen 10-100fach höheren $k_{cat}$s-Wert besitzen als E.coli-AP. Unter den Säuger-APs zeigen die APs aus dem Rinderdarm (bIAP) die höchsten spezifischen Aktivitäten. Diese Eigenschaft machen die bIAPs attraktiv für biochemische Anwendungen wie z.B. der Einsatz entsprechender Enzymkonjugate als diagnostisches Reagenz oder zur Dephosphorylierung von DNA. Die Existenz verschiedener Alkalischer Phosphatasen aus dem Rinderdarm mit unterschiedlich hohen spezifischen Aktivitäten ist in EP 0 955 369 bzw. Manes et al. (1998), J. Biol. Chem. 273 No. 36, 23353-23360, beschrieben. Bislang ist eine rekombinante Expression von eukaryontischen niederaktiven (bis 3000 U/mg) Alkalischen Phosphatasen in verschiedenen eukaryontischen Zelllinien wie z.B. CHO-Zellen (bIAP I/WO 93/18139; Weissig et al. 1993, Biochem J. 260, 503-508), COS-Zellen (humane Placenta-AP/Berger et al., 1987 Biochemistry 84, 4885-4889) oder Baculovirus-Expressionssystem (humane Placenta-AP/Davis et al. 1992, Biotechnology 10, 1148-1150) beschrieben. Auch ist die Expression von höher aktiven AP's (spez. Aktivität >3000 U/mg) aus dem Rinderdarm in CHO-Zellen beschrieben (bIAP II, III und IV/Manes et al. 1998, J. Biol. Chem. 273 No. 36, 23353-23360). Nachteil der Expression von Alkalischen Phosphatasen in diesen Expressionssystemen ist jedoch die geringe Expressionsleistung, die die rekombinante Herstellung von eukaryontischen Alkalischen Phosphatasen nicht wirtschaftlich macht.

Eine Expression von eukaryontischen Alkalischen Phosphatasen in prokaryontischen Expressionswirten wie z.B. E. coli ist zwar prinzipiell möglich (humane Placenta-AP/Beck and Burtscher, 1994 Protein Expression and Purification 5, 192-197), jedoch weisen die in Prokaryonten exprimierten Alkalischen Phosphatasen keine Glykosylierung auf, die insbesondere bei der Herstellung von Enzymkonjugaten je nach Konjugationsverfahren essentiell ist.

Eine häufige Anwendung der Alkalischen Phosphatase als Enzymkonjugat ist ein Komplex mit einem Antikörper. Hierbei wird die Alkalische Phosphatase mit einem Antikörper konjugiert, der gegen ein bestimmtes Antigen gerichtet ist. Dieses Antigen wird zunächst von einem an die Gefäßwand immobilisierten Antikörper, der ein anderes Epitop an dem Ziel-Antigen erkennt, als das Antikörper-AP-Konjugat, in einer ersten Reaktion gebunden. Dieser Antikörper-Antigen-Komplex wird dann in einer zweiten Reaktion durch die Bindung des Antikörper-AP-Konjugats nachgewiesen. Bei derartigen Tests kommt es immer wieder zu falsch positiven Ergebnissen, hervorgerufen durch eine unspezifische Bindung des Antikörper-AP-Konjugates mit der Gefäßwand oder dem ersten Antikörper. Diese Störungen lassen sich durch Zugabe eines Konjugates mit einer inaktiven oder schwach aktiven AP-Mutante im Überschuss als Entstörprotein vermeiden. Um sehr spezifisch als Entstörprotein wirken zu können ist jedoch neben der geringen oder fehlenden Aktivität auch eine weitgehend gleiche Tertiär- und Quartiärstruktur der AP-Mutanten zwingend Voraussetzung.

Demzufolge liegt der vorliegenden Erfindung die Aufgabe zugrunde, durch gezielte Mutagenese Mutanten der Alkalischen Phosphatase als Entstörprotein zu erzeugen, die nur sehr schwach oder gänzlich inaktiv sind, in Ihrer Aminosäuresequenz aber nur geringfügig und in der Tertiär- und Quatiärstruktur möglichst nicht verändert sind. Desweiteren ist eine Aufgabe der Erfindung, ein widerstandsfähiges und stabiles Expressionsverfahren zur Herstellung glykosylierter eukaryontischer Alkalischer Phosphatase- Mutanten zu entwickeln, das zudem auf Grund hoher Expressionsleistung eine wirtschaftliche Herstellung einer entsprechenden Alkalischen Phosphatase-Mutante ermöglicht.

Gegenstand der vorliegenden Erfindung sind Mutanten der eukaryontischen Alkalischen Phosphatase, wobei

- die zu mutierende Sequenz SEQ ID NO.: 2 ist, und
- die Mutante eine um mindestens den Faktor 100 verringerte Aktivität im Vergleich zum Wildtyp aufweist, und
- die Mutante eine oder mehrere der nachfolgend genannten Mutationen aufweist, und wobei die Position der Mutation relativ zur Position in SEQ ID NO.: 2 definiert ist:

| | |
|---|---|
| Ser92: | gegen Ala oder Gly |
| His320: | gegen Asn oder Phe |
| Gly 322: | gegen Pheoder Lys, |

Unter Mutation der Aminosäuresequenz versteht man den Austausch der natürlich vorkommenden Aminosäure an der gewünschten Position gegen eine beliebig andere Aminosäure, die die Faltung in die korrekte Tertiär- und Quartiärstruktur nicht behindert, jedoch nicht funktionell sind.

Die zu mutierende Sequenz (Wildtyp) kann beispielsweise eine humane Darm-AP sein oder eine humane Plazenta-AP sein. Außerdem kommen als Wildtyp-AP eine niederaktive oder eine hochaktive AP aus Rinderdarm erfindungsgemäß in betracht. All diese Enzyme sind zu mindestens 77% homolog zur SEQ ID NO.: 2. Die Homologie wurde bestimmt mit der Software "Open VMS Vax Version V6.2";(Copyright (c) 1982-2001, Genetics Computer Group, Inc. ; A wholly owned subsidiary of Oxford Molecular Group, Inc. All rights reserved. Published research assisted by this software should cite: Wisconsin Package Version 10.2, Genetics Computer Group (GCG), Madison, Wisc.)

Die Angabe der Position der zu mutierenden Aminosäure bezieht sich auf die Aminosäuresequenz der native Alkalische Phosphatase gemäß SEQ ID NO.: 2, ohne Signalsequenz. Die ausgewiesenen Aminosäurepositionen sind jedoch auch auf andere Alkalische Phosphatasen aus Rind oder anderen Organismen übertragbar; die Austausche betreffen Aminosäuren mit hoch konservierten Funktionen innerhalb des Proteins und somit muß nur die Position nach der betreffenden Aminosäuresequenz angepasst werden.

Gezielte Mutagenese der DNA-Sequenz bedeutet, daß einzelne oder mehrere Codons via PCR-Mutagenese verändert werden. Dabei werden so wenige Veränderungen wie nötig im Basentriplet, im günstigsten Fall nur eine Base, des codonoptimierten Gens gemäß SEQ ID NO.: 3 geändert.

Bevorzugt sind erfindungsgemäß Mutanten der eukaryontischen Alkalischen Phosphatase gemäß Anspruch 1, wobei die Mutante eine oder mehrere der nachfolgend genannten Mutationen aufweist, und wobei die Position der Mutation relativ zur Position in SEQ ID NO.: 2 definiert ist:

| | |
|---|---|
| Ser92: | gegen Ala oder Gly |
| His320. | gegen Asn oder Phe |
| Gly 322. | gegen Phe oder Lys |

Erfindungsgemäß bevorzugt sind die oben genannten Mutanten der eukaryontischen Alkalischen Phosphatase, wobei das Enzym nach der Wildtyp-Sequenz eine spezische Aktivität von über 7000 U/mg aufweist. Weiterhin bevorzugt ist als Gensequenz eine DNA-Sequenz, die basierend auf dem Gen, welches für eine eukaryontische Alkalische Phosphatase-Mutante mit einer spezifischen Aktivität von über 7000 U/mg codiert, an wenigen Positionen gezielt mutiert wurde, so daß die so entstandene DNA-Sequenz für eine an einer oder wenigen Positionen veränderte Aminosäuresequenz der eukaryontischen Alkalische Phosphatase-Mutanten kodiert, wobei die Mutationen zu einer starken bis totalen Reduzierung der spezifischen Aktivität führen.

Besonders bevorzugt sind die erfindungsgemäßen Mutanten der eukaryontischen Alkalischen Phosphatase, wobei die Mutante eine um mindestens den Faktor 1000 verringerte AP-Aktivität im Vergleich zum Wildtyp-Enzym aufweist. Darüberhinaus bevorzugt sind Mutanten der erfindungsgemäßen eukaryontischen Alkalischen Phosphatase, wobei die Mutante eine um mindestens den Faktor 10000 verringerte Aktivität im Vergleich zum entsprechenden Wildtyp-Enzym aufweist. Ganz besonders bevorzugt sind erfindungsgemäß solche Mutanten, bei denen die Erniedrigung der spezifischen Aktivität unterhalb der Nachweisgrenze liegt (bestimmt gemäß des in Beispiel 4 aufgeführten Aktivitätstests).

Erfindungsgemäß haben sich die Aminosäurepositionen Asp316/His320/His432 (Bindungspartner von Zinkatom 1), Asp42/Asp357/ His358 (Bindungspartner von Zinkatom 2), Ser155/Glu311 (Bindungspartner des Magnesiumatoms), Ser92 (Hydroxylgruppe wird deprotoniert für nukleophilen Angriff auf das Substrat) (Ma and Kantrowitz (1994), J. Biol. Chem, 16 pp. 31614-31619; Ma et al. (1995), Protein Science, 4, pp. 1498-1506; Kimura and Kikuta (2000) JBIC 5, pp. 139-155; Stec et al. (2000), JMB 299 pp. 1303-1311) sowie Gly322 (wichtig für die spezifische Aktivität; EP 0 955 369) als geeignet erwiesen. Erfindungsgemäß kommen hier die oben beschriebenen Positionen als Einzelmutanten oder aber auch in allen möglichen Kombinationen der oben beschriebenen Positionen als Doppel-, Dreifach oder Vielfachmutanten in betracht. Besonders bevorzugt werden die mutierten Aminosäure-Sequenzen gemäß SEQ ID NO's: 4-7, wobei SEQ ID NO.: 4 eine Einzelmutante in Position 92 (Ser92Ala), SEQ ID NO.: 5 eine Einzelmutante in Position 322 (Gly322Phe), SEQ ID NO.: 6 eine Doppelmutante in den Positionen 320 und 322 (His320Asn/ Gly322Phe) und SEQ ID NO.: 7 eine Dreifachmutante in den Positionen 92, 320 und 322 (Ser92Ala/His320Asn/Gly322Phe) darstellen. Die dazugehörenden DNA-Sequenzen sind in SEQ ID NO.: 8-11 dargestellt.

Gegenstand der vorliegenden Erfindung ist ferner eine DNA, welche für die oben beschriebenen erfindungsgemäßen Mutanten kodiert. Desweiteren sind Gegenstand der vorliegenden Erfindung Vektoren, welche die erfindnungsgemäße Nukleinsäuresequenz enthalten. Insbesondere sind dies solche eine Nukleinsäuresequenz enthaltende Vektoren, wobei die Nukleinsäuresequenz ausgewählt ist aus den SEQ ID NO's: 4-7. Geeignete Vektoren sind dem Fachmann bekannt, wie beispielsweise pPICZαA, B, C;pPICZ, pPICZ-E, pPICZα-E; pPIC6, pPIC6αA, B, C; pGAPZ, pGAPZαA, B, C; pPIC9; pPIC9K, pPIC3.5, pPIC3.5K, pAO815, pMET, pMETαA, B, C;pYES-Dest52, pYES2.1/V5-His-TOPO, pYC2-E, pYES2.1-E; Yes-Vektoren, pTEF1/Zeo, pTEF1/Bsd, pNMT-TOPO (z.B. Invitrogen). Kloniert sind die entsprechenden Gensequenzen beispielsweisse in die Vektoren pPICZαA bzw. pPIC9K, die kommerziell erhältlich sind (Invitrogen), und die die erfindungsgemäßen Gensequenzen gemäß SEQ ID NO's: 8-11 enthalten, die unter Kontrolle des AOX1-Promotors stehen. Beispielhaft für die erfindungsgemäß hergestellten Vektoren seien die Vektoren pNaAP31-1 (Fig. 1) und pNaAP31-2 (Fig. 2), die die Gensequenz gemäß SEQ ID NO.: 8 jeweils kloniert in pPICZαA (pNaAP31-1) und pPIC9K (pNaAP31-2) besitzen, angeführt.

Die Vektoren pNaAP31-1 und pNaAP31-2 sind gleichermaßen für die erfindungsgemäß hergestellten Vektoren relevant, da der letztendliche Produktionsklon Kopien von beiden Vektoren enthalten kann.

Ein erfindungsgemäß erhaltender Expressionsvektor wird bevorzugt in verschiedene Stämme von Hefen wie z.B. Pichia pastoris transformiert und stabil in das Genom integriert. Die stabile Integration in das Hefegenom hat insbesondere den Vorteil, daß bei der späteren Produktion der beispielsweise schwach aktiven oder inaktiven Alkalischen Phosphatase-Mutanten in großvolumigen Fermentern kein Selektionsdruck benötigt wird. Stabile Integration in das Genom bedeutet, daß der Expressionsvektor über homologe Rekombination in das Genom von z.B. Pichia pastoris eingebaut wird und somit als fester Bestandteil des Hefegenoms von Generation zu Generation weitervererbt wird (Cregg, J.M. et al., Mol. Cell. Biol. 5 (1985), 3376-3385).

Gegenstand der Erfindung ist des weiteren ein Wirtstamm transformiert mit einem der erfindungsgemäßen Vektoren. Als Hefe-Wirt sind insbesondere methylotrophe Hefen, z.B. Hefe Pichia pastoris, Hansenula polymorpha, Saccharomyces cerevisiae, Yarrowia lipolytica oder Schizosaccharomyces pombe geeignet. Insbesondere bevorzugt wird als Wirtstamm Pichia pastoris verwendet. Besonders bevorzugt ist der Pichia pastoris X-33 Stamm transformiert mit einem der beschriebenen Vektoren.

Gegenstand der Erfindung ist ebenfalls ein Verfahren zur Herstellung der erfindungsgemäßen Mutante der eukaryontischen Alkalischen Phosphatase in Hefezellen, umfassend die Schritte: a) Klonierung einer erfindungsgemäßen Gensequenz in unterschiedlichen Vektoren, b) Transformation der Hefe, c) Expression und d) Reinigung der Alkalischen Phosphatase, **dadurch gekennzeichnet, daß**

- ein erster Vektor ein Resistenzgen gegen einen ersten Selektionsmarker aufweist,
- Transformanten, die das Resistenzgen und die gewünschte Gensequenz in das Genom integriert haben, durch Wachstum auf Nährmedium mit einer geringen Konzentration an erstem Selektionsmarker selektioniert werden,
- die Genkopienzahl durch Mehrfachtransformation erhöht wird, wobei Mehrfachtransformanten durch Wachstum auf Nährmedium mit erhöhtem Selektionsdruck selektioniert werden,
- ein zweiter Vektor, welcher ein Resistenzgen gegen einen zweiten Selektionsmarker aufweist, zugegeben wird,
- die Genkopienzahl durch Mehrfachtransformation mit dem zweiten Vektor erhöht wird, wobei Mehrfachtransformanten durch Wachstum auf Nährmedium mit erhöhtem Selektionsdruck selektioniert werden, und
  solche Klone selektioniert werden, die mehrere Kopien der Gensequenz und der Selektionsmarker-Resistenzgene stabil in das Genom integriert haben.

Bevorzugt werden in dem erfindungsgemäßen Verfahren methylotrophe Hefezellen verwendet. Insbesondere bevorzugt wird als Hefezelle Pichia pastoris verwendet.

Es ist ferner für das erfindungsgemäße Verfahren bevorzugt, daß als Vektor ein solcher verwendet, der einem Vektor entspricht, welcher ausgewählt ist aus den folgenden Vektoren: pPICZαA, B, C;pPICZ, pPICZ-E, pPICZα-E; pPIC6, pPIC6αA, B, C; pGAPZ, pGAPZαA, B, C; pPIC9; pPIC9K, pPIC3.5, pPIC3.5K, pAO815, (Invitrogen).

Die Erhöhung der Kopienzahl der mutierten Gensequenz in der methylotrophen Hefe wurde durch Mehrfachtransformation bei gleichzeitiger Erhöhung des Selektionsdruckes mit einem geeigneten Selektionsmarker, z.B. einem Antibiotikum wie Zeocin® bzw. Geneticin (G418) erreicht, wonach nur Klone lebensfähig sind, die mehrere Kopien des Expressionsvektors stabil in das Genom integriert haben. Um gegen höhere Konzentrationen des als Selektionsmarker verwendeten Antibiotikums resistent zu sein, ist es erforderlich, daß die Klone vermehrt Resistenzproteine produzieren. Dies kann beispielsweise durch eine multiple Integration des Expressionsvektors erfolgen, der neben der Expressionskassette für die jeweilige AP-Mutante, wie z.B. die Alkalische Phosphatase-Mutante Ser92Ala, auch das Resistenzgen für das als Selektionsmarker verwendete Antibiotikum enthält.

Die Aufgabe Alkalische Phosphatase-Mutanten in einem widerstandsfähigen und stabilen Expressionsverfahren mit hoher Expressionsleistung und zugleich in wirtschaftlichen Ausbeuten herzustellen, konnte durch das nachfolgend beschriebene Verfahren erreicht werden.

Die gezielte Mutagenese zur Herstellung der erfindungsgemäßen Mutanten wurde wie folgt durchgeführt:

Basierend auf dem Gen, das für eine Alkalische Phosphatase-Mutante aus Rind kodiert und das synthetisch hergestellt wurde, wurden zueinander komplementäre oder überlappend komplementäre Oligonukleotide entworfen, die in einer oder mehreren Basenpositionen gegenüber SEQ ID NO.: 3 verändert waren. Einer dieser Primer wurde anschließend als Partner mit dem nachfolgend beschriebenen 5'- bzw. 3'-primer in die PCR-Reaktion eingesetzt und somit das AP-Gen in zwei Teilstücken mit der oder den gewünschten Basenaustauschen amplifiziert.

Die zwei Teilstücke wurden anschließend mittel Agarosegelelektrophorese analysiert, die Produkte mit der erwarteten Länge aus dem Gel mittels QIAquick Gel Extraction Kit (Qiagen) isoliert und in einer weiteren PCR-Reaktion zu dem kompletten Genprodukt synthetisiert. Die PCR-Reaktion wurde dabei in den ersten 5 Zyklen ohne Zugabe der Primer am 5'-Ende und am 3'-Ende des gesamten Gens durchgeführt, so daß zunächst aus den zwei Teilstücken wenige Fragmente des Genproduktes der erwarteten Länge entstehen. Die Annealing-Temperatur richtet sich dabei nach der Schmelztemperatur des Überlappungsbereich. Danach wurden die endständigen Primer zugegeben und die Annealing-Temperatur entsprechend der Annealing-Temperatur des Primers mit der geringsten Schmelztemperatur erhöht. In weiteren 25 Zyklen wurde danach das Genfragment der erwarteten Länge hochamplifiziert.

Der PCR-Ansatz wurde mittels Agarosegelelektrophorese analysiert und das Genfragment mit der erwarteten Größe isoliert (QIAquick Gel Extraction Kit/Qiagen).

Die Klonierung eines entsprechenden PCR Fragmentes, die Transformation in Pichia pastoris sowie die Expression der entsprechenden AP-Mutante sind in Beispiel 2 beschrieben.

Die rekombinanten Alkalischen Phosphatase-Mutanten können durch Extraktionsmethoden, die dem Fachmann prinzipiell bekannt sind, aus der Biomasse isoliert werden z.B. nach "Protein Purification", Springer-Verlag, Herausg. Robert Scopes (1982). Durch geeignete chromatographische Methoden, wie insbesondere unter Verwendung von hydrophoben Säulenmaterialien und eines Kationenaustauschers, wird ein bandenreines Produkt erzielt.

Mit der vorliegenden Erfindung wird somit erstmals ein Verfahren beschrieben, daß eine wirtschaftliche Herstellung von rekombinanten Alkalischen Phosphatase-Mutanten aus Säugetieren, wie z.B. Rinderdarm, in Hefe ermöglicht, die sehr stark reduzierte oder keine AP-Aktivität mehr besitzen, ansonsten aber in ihren Eigenschaften der rekombinanten Alkalischen Phosphatase-Mutanten aus dem Rinderdarm entsprechen. Diese Mutanten können insbesondere als Entstörprotein in immunologischen Testverfahren, bei denen AP als Label fungiert, wie MTP-ELISA eingesetzt werden.

<u>Abbildungen</u>

Abbildung 1

Plasmidkarte von Expressionsvektor pNaAP31-1 mit der mutierten Gensequenz gemäß SEQ ID NO.: 8 in pICZαA (Invitrogen).

Abbildung 2

Plasmidkarte von Expressionsvektor pNaAP31-1 mit der mutierten Gensequenz gemäß SEQ ID NO.: 8 in pIC9K (Invitrogen).

<u>Erläuterungen zu den Sequenzprotokollen SEQ ID NO's 1-21</u>

| | |
|---|---|
| SEQ ID NO.: 1 | Native DNA-Sequenz kodierend für die hochaktive AP aus Rind, ohne Signalsequenz |
| SEQ ID NO.: 2 | Aminosäuresequenz der hochaktiven AP aus Rind |
| SEQ ID NO.: 3 | DNA-Sequenz des synthetischen Gens kodierend für eine hochaktive AP, stromaufwärts der kodierenden Sequenz befindet sich die Restriktionsschnittstelle EcoRI, stromabwärts die Restriktionsschnittstelle Asp 718 |
| SEQ ID NO.: 4 | Aminosäuresequenz der AP-Einzelmutante Ser 92 Ala (Wildtyp: hochaktive AP aus Rind) |
| SEQ ID NO.: 5 | Aminosäuresequenz der AP-Einzelmutante Gly 322 Phe (Wildtyp: hochaktive AP aus Rind) |
| SEQ ID NO.: 6 | Aminosäuresequenz der AP-Doppelmutante His 320/Gly 322 Phe (Wildtyp: hochaktive AP aus Rind) |
| SEQ ID NO.: 7 | Aminosäuresequenz der AP-Dreifachmutante Ser 92 Ala/His 320/Gly 322 Phe (Wildtyp: hochaktive AP aus Rind) |
| SEQ ID NO.: 8 | DNA-Sequenz des synthetischen Gens kodierend für die AP-Einzelmutante Ser 92 Ala mit EcoRI- bzw. Asp 718-Schnittstelle, stromaufwärts bzw. stromabwärts der kodierenden Sequenz |
| SEQ ID NO.: 9 | DNA-Sequenz des synthetischen Gens kodierend für die AP-Einzelmutante Gly 322 Phe mit |

| | |
|---|---|
| | EcoRI- bzw. Asp 718-Schnittstelle, stromaufwärts bzw. stromabwärts der kodierenden Sequenz |
| SEQ ID NO.: 10 | DNA-Sequenz des synthetischen Gens kodierend für die AP-Doppelmutante His 320 Asn/Gly 322 Phe mit EcoRI- bzw. Asp 718-Schnittstelle, stromaufwärts bzw. stromabwärts der kodierenden Sequenz |
| SEQ ID NO.: 11 | DNA-Sequenz des synthetischen Gens kodierend für die AP-Dreifachmutante Ser 92 Ala/His 320 Asn/Gly 322 Phe mit EcoRI- bzw. Asp 718-Schnittstelle, stromaufwärts bzw. stromabwärts der kodierenden Sequenz |
| SEQ ID NO's.: 12-21 | DNA-Sequenzen die als Primer dienten |

Abkürzungen

| | |
|---|---|
| YPD: | Yeast Peptone Dextrose |
| YPDS: | Yeast Peptone Dextrose Sorbitol |
| BMGY: | Buffered Glyerol-complex medium |
| BMMY: | Buffered Methanol-complex medium |
| MTP: | Microtiterplatten |

Beispiel 1:

Mutagenese der synthetischen DNA-Sequenz, die für die Alkalische Phosphatase-Mutanten aus Rind kodiert.

Zur Mutagenese des oder der gewünschten Basentriplets via PCR-Reaktion wurden Oligonukleotide entworfen, die eine entsprechend geänderte Basensequenz besitzen und zueinander komplementär bzw zueinander teilweise überlappend komplementär sind. Diese Oligonukleotide wurden anschließend als entsprechender Partner zu dem 5'-Primer 5'-hAP gemäß SEQ ID NO.: 12 bzw. dem 3'-Primer 3'-hAP gemäß SEQ ID NO.: 13, die jeweils mit dem 5'-Ende bzw. dem 3'-Ende des Gens, das für die Alkalische Phosphatase-Mutanten aus Rind kodiert, hybridisieren, eingesetzt. Somit wurden in einer ersten Reaktion die mutierte Gensequenz in zwei Teilstücken amplifiziert, wobei das erste Teilstück die Mutation am 3'-Ende und das zweite Teilstück die Mutation am 5'-Ende trägt und eine kurze Basensequenz am 3'-Ende vom ersten Teilstück identisch ist mit einer kurzen Basensequenz des 5'-Ende des zweiten Teilstückes.

In einer zweiten PCR-Reaktion wurden dann diese zwei Teilstücke zu dem Voll-Längengenprodukt fusioniert. Dazu wurde die PCR-Reaktion zunächst ohne die 5'-hAP und 3'-hAP Primer gemäß SEQ ID NO.: 12 und 13 gestartet und 5 Zyklen durchlaufen. Dabei entstehen wenige Moleküle des Voll-Längengenprodukt, die Annealingtemperatur bei diesen 5 Zyklen richtet sich nach der Schmelztemperatur des überlappenden Bereiches der beiden Teilstücke. Anschließend werden die die 5'- und 3'-Primer gemäß SEQ ID NO.: 12 und 13 zugegeben, die Annealingtemperatur der Schmelztemperatur des Primers mit der niedrigeren Schmelztemperatur angepasst und das Voll-Längengenprodukt in weiteren 25 Zyklen amplifiziert.

Mutagenese zur Generierung der Einzelmutante Ser92Ala:

Zur Generierung der Einzelmutante Ser92Ala wurde das Basentriplet in Position 274-276, bezogen auf das Basentriplet TTG gemäß SEQ ID NO.: 3, das für die erste Aminosäure der hochaktiven Alkalischen Phosphatase aus Rind kodiert, von TCT in GCT mutiert. Dazu wurden die zueinander teilweise komplementären Primer 5'-S92A gemäß SEQ ID NO.: 14 und 3'-S92A gemäß SEQ ID NO.: 15 entworfen. Die erste PCR-Reaktion wurde anschließend mit den Primerpaaren 5'-hAP und 3'-S92A sowie 5'-S92A und 3'-hAP getrennt voneinander mit der Gensequenz gemäß SEQ ID NO.: 3 als template gestartet, so daß die mutierte Gensequenz zunächst in zwei Teilstücken amplifiziert wurde. Die Teilstücke wurden mittels Agarosegel analysiert und aus dem Agarosegel (QIAquick Gel Extraction Kit/Qiagen) isoliert und anschließend in die zweite PCR-Reaktion eingesetzt. In dieser zweiten PCR-Reaktion wurden die beiden Teilstücke zum Voll-Längenprodukt wie oben beschrieben fusioniert. Die so entstandene mutierte Gensequenz wurde entsprechende PCR-cloning Vektoren (PCR cloning Kit - blunt end/Roche Diagnostics) kloniert und mittels Restriktionsanalyse und Sequenzierung überprüft.

Mutagenese zur Generierung der Einzelmutante Gly322Phe

Zur Generierung der Einzelmutante Gly322Phe wurde das Basentriplet in Position 964-966, bezogen auf das erste Basentriplet TTG gemäß SEQ ID NO.: 3, das für die erste Aminosäure der hochaktiven Alkalischen Phosphatase aus Rind kodiert, von GGT in TTT mutiert. Dazu wurden die zueinander teilweise komplementären Primer 5'-G322F

gemäß SEQ ID NO.: 16 und 3'-G322F gemäß SEQ ID NO.: 17 entworfen. Die erste PCR-Reaktion wurde dann mit dem Primerpaaren 5'-hAP und 3'-G322F sowie 5'-G322F und 3'-hAP getrennt voneinander mit der Gensequenz gemäß SEQ ID NO.: 3 als template gestartet, so daß die mutierte Gensequenz zunächst in zwei Teilstücken amplifiziert wurde. Die Teilstücke wurden mittels Agarosegel analysiert und aus dem Agarosegel (QIAquick Gel Extraction Kit/Qiagen) isoliert und anschließend in die zweite PCR-Reaktion eingesetzt. In dieser zweiten PCR-Reaktion wurden die beiden Teilstücke zum Voll-Längenprodukt wie oben beschrieben fusioniert. Die so entstandene mutierte Gensequenz wurde entsprechende PCR-cloning Vektoren (PCR cloning Kit - blunt end/Roche Diagnostics) kloniert und mittels Restriktionsanalyse und Sequenzierung überprüft.

Mutagenese zur Generierung der Doppelmutante His320Asn/Gly322Phe

Zur Generierung der Doppelmutante His320Asn/Gly322Phe wurde die Basentriplets in den Positionen 958-960 bzw. 964-966, bezogen auf das erste Basentriplet TTG gemäß SEQ ID NO.: 3, das für die erste Aminosäure der hochaktiven Alkalischen Phosphatase aus Rind kodiert, von CAT in AAT bzw. GGT in TTT mutiert. Dazu wurden die zueinander teilweise komplementären Primer 5'-H320N/G322F gemäß SEQ ID NO.: 18 und 3'-H320N/G322F gemäß SEQ ID NO.: 19 entworfen. Die erste PCR-Reaktion wurde anschließend mit den Primerpaaren 5'-hAP und 3'-H320N/G322F sowie 5'-H320N/G322F und 3'-hAP getrennt voneinander mit der Gensequenz gemäß SEQ ID NO.: 3 als template gestartet, so daß die mutierte Gensequenz zunächst in zwei Teilstücken amplifiziert wurde. Die Teilstücke wurden mittels Agarosegel analysiert und aus dem Agarosegel (QIAquick Gel Extraction Kit/Qiagen) isoliert und anschließend in die zweite PCR-Reaktion eingesetzt. In dieser zweiten PCR-Reaktion wurden die beiden Teilstücke zum Voll-Längenprodukt wie oben beschrieben fusioniert. Die so entstandene mutierte Gensequenz wurde entsprechende PCR-cloning Vektoren (PCR cloning Kit - blunt end/Roche Diagnostics) kloniert und mittels Restriktionsanalyse und Sequenzierung überprüft.

Generierung der Triplemutante Ser92Ala/His320Asn/Gly322Phe

Die Generierung der Triplemutante Ser92Ala/His320Asn/Gly322Phe erfolgte durch Kombination der Einzelmutante Ser92Ala und der Doppelmutante His320Asn/Gly322Phe. Dazu wurden beide mutierten Gensequenzen, jeweils kloniert in PCR cloning Vektoren (PCR cloning Kit - blunt end/Roche Diagnostics) getrennt voneinander mit den Restriktionsendonukleasen MunI und Asp718 geschnitten, der Restriktionsansatz mittels Agarosegelelektrophorese aufgetrennt. MunI schneidet zwischen den Positionen der Triplets von Ser92 und His320. Von der Einzelmutante Ser92Ala wurde das ca. 3700 bp lange Vektorfragment isoliert und von der Doppelmutante His320Asn/Gly322Phe wurde das ca 900 bp grosse Fragment des 3'-Bereiches der mutierten Gensequenz aus dem Agarosegel isoliert und diese beiden Fragmente im nächsten Schritt miteinander ligiert. Die so entstandene Gensequenz wurde durch Sequenzierung überprüft.

Beispiel 2:

Klonierung der mutierten Gensequenzen in den Expressionsvektor pPICZαA für Pichia pastoris

Die überprüften mutierten Gensequenzen wurden durch Restriktion mit den Restriktionsendonukleasen EcoRI und Asp718 aus den PCR-Klonierungsvektoren ausgeschnitten, der Restriktionsansatz mittels Agarosegelelektrophorese aufgetrennt und die ca. 1480 bp langen Fragmente aus dem Agarosegel (QIAquick Gel Extraction Kit/Qiagen) isoliert. Anschließend wurden die mutierten Gensequenzen mit einem ebenfalls mit EcoRI und Asp718 linearisierten Vektorfragment von den Expressionsvektor pPICZαA ligiert. Die notwendigen Restriktionsendonukleaseschnittstellen für EcoRI und Asp718 wurden durch die Primer 5'-hAP bzw. 3'-hAP in die mutierten Gensequenzen eingebracht, die die Erkennungssequenzen für Restriktionsendonuklease EcoRI bzw. Asp718 stromaufwärts bzw. stromabwärts der kodierenden Sequenz besitzen. Die so entstandenen Expressionsvektoren für Alkalische Phosphatase-Mutanten wurden wie folgt bezeichnet: pNaAP31-1(Ser92Ala), pNaAP43-1 (Gly322Phe), pNaAP51-1 (His320Asn/Gly322Phe) und pNaAP6-1 (Ser92Ala/His320Asn/Gly322Phe).

In diesem Vektor stehen die mutierten Gensequenzen unter Kontrolle des AOX 1-Promotors (Promotor für die Alkoholoxidase 1 aus Pichia pastoris), mit Methanol induzierbar und wird im korrekten Leserahmen hinter das Signalpeptid des α-Faktors aus Saccharomyces cerevisiae kloniert. Das so insertierten Genfragment wurde dann mittels Restriktionsanalyse und Sequenzierung auf fehlerfreie Basensequenz untersucht. Die so entstandene Expressionsvektoren, die jeweils eine der mutierten Gensequenzen gemäß SEQ ID NO's.: 8-11, die für eukaryontische Alkalische Phosphatase-Mutanten kodieren, sind beispielhaft an pNaAP31-1 (s. Fig. 1) gezeigt.

Transformation der Expressionsvektoren mit mutierten Gensequenzen in Pichia pastoris

Zur Transformation der Expressionsvektoren mit mutierten Gensequenzen in Pichia pastoris X-33 mit anschließender Integration in das Genom wurden die Vektoren zunächst mit SacI (Roche Diagnostics GmbH) linearisiert. Die Transformation wurde mittels Elektroporation mit dem Gene Pulser II (Biorad) durchgeführt.

Hierfür wurde eine Kolonie von Pichia pastoris Wildtypstamm in 5 ml YPD-Medium (Invitrogen) angeimpft und bei 30°C über Nacht unter Schütteln inkubiert. Die Übernachtkultur wurde anschließend 1:2000 in 200 ml frisches YPD-Medium (Invitrogen) überimpft und über Nacht bei 30°C unter Schütteln inkubiert, bis eine $OD_{600}$ von 1,3 - 1,5 erreicht war. Die Zellen wurden abzentrifugiert (1500 x g / 5 Minuten) und das Pellet in 200 ml eiskaltem, sterilen Wasser (0°C) resuspendiert. Die Zellen wurden wiederum abzentrifugiert (1500 x g/5 Minuten) und in 100 ml eiskaltem, sterilen Wasser (0°C) resuspendiert. Die Zellen wurden wiederum abzentrifugiert, in 10 ml eiskaltem (0°C) 1 M Sorbitol (ICN) resuspendiert. Die Zellen wurden wiederum abzentrifugiert, in 0,5 ml eiskaltem (0°C) 1 M Sorbitol (ICN) resuspendiert. Die so gewonnenen Zellen wurden auf Eis gehalten und sofort zur Transformation eingesetzt.

80 µl der Zellen wurden mit ca. 1 µg linearisierter Expressions-Vektor-DNA versetzt und der gesamte Ansatz in eine eiskalte (0°C) Elektroporationsküvette transferiert und weitere 5 Minuten auf Eis inkubiert. Anschließend wurde die Küvette in den Gene Pulser II (Biorad) überführt und die Transformation bei 1 kV, 1 kΩ und 25µF durchgeführt. Nach der Elektroporation wurde der Ansatz mit 1 ml 1M Sorbitol (ICN) versetzt und anschließend 100 bis 150 µl auf eine YPDS-Agarplatte (Invitrogen) mit 100µg/ml Zeocin® (Invitrogen) ausplattiert. Die Platten wurden anschließend 2-4 Tage bei 30 °C inkubiert.

Die Klone wurden auf Raster-MD (= minimal Dextrose)-Platten überimpft und weiter analysiert. Gewachsene Klone wurden gepickt, in 20 µl steriles Wasser resuspendiert, mit 17,5 U Lyticase (Roche Diagnostics GmbH) aufgeschlossen (1 Stunde, 37°C) und direkt mittels PCR auf die korrekte Integration der Expressionskassette mit entsprechender mutierter Gensequenz untersucht.

Klone, die die komplette Expressionskassette bei der Transformation in das Genom integriert haben, wurden dann in Expressionsversuchen eingesetzt.

Beispiel 3:

Expression der Alkalischen Phosphatase-Mutanten

Positive Klone wurden in 3 ml BMGY-Medium (Invitrogen) angeimpft und über Nacht bei 30°C unter Schütteln inkubiert. Anschließend wurden die OD bei 600 nm bestimmt und so in 10ml BMMY-Medium (Invitrogen) überimpft, daß eine $OD_{600}$ von 1 resultierte. Das BMMY-Medium (Invitrogen) enthält Methanol (Mallinckrodt Baker B.V.), das die Expression der Alkalische Phosphatase-Mutanten über den AOX 1-Promotor induziert.

Die Schüttelkolben wurden bei 30°C unter Schütteln inkubiert, alle 24 Stunden Proben gezogen, die $OD_{600}$ bestimmt, ein Aktivitätstest auf Expression der Alkalische Phosphatase-Mutanten durchgeführt und jeweils mit 0,5% Methanol (Mallinckrodt Baker B.V.) zur weiteren Induktion nachgefüttert. Die Expressionsversuche liefen über 96 Stunden.

Beispiel 4:

Test auf Aktivität der Alkalische Phosphatase-Mutanten

Je 500 µl wurden der Expressionskultur gemäß Beispiel 3 entnommen, die $OD_{600}$ bestimmt und die Zellen abzentrifugiert. Der Überstand wurde aufbewahrt und das Zellpellet wurde in einer der $OD_{600}$ entsprechenden Menge Y-PER™ (50 bis 300 µl/Pierce) zur Lyse resuspendiert und 1 Stunde bei Raumtemperatur geschüttelt. Anschließend wurde das Lysat zur Abtrennung von Zelltrümmern abzentrifugiert (15000 x g/5 Minuten) und der Überstand in frische Reaktionsgefäße überführt. 5 µl des Lysates wurden dann im Aktivitätstest eingesetzt.

Der Aktivitätstest funktioniert nach folgendem Prinzip:

$$\text{4-Nitrophenylphosphat} + H_2O \xrightarrow{AP} \text{4-Nitrophenol} + P_i$$

Gemessen wird die Absorptionszunahme bei 405 nm.

3 ml Diethanolamin-Puffer (1 mol/L Diethanolamin (Merck) pH 9,8, 0,5 mmol/L $MgCl_2$ (Riedel de Haen)) wurden

mit 50 µl 4- Nitrophenylphosphatlösung (0,67 Mol/L 4- Nitrophenylphosphat, Na-Salz (Roche Diagnostics GmbH)) versetzt und der Ansatz auf 37°C temperiert. Anschließend wurde die Reaktion durch Zugabe von 5 µl Lysat gestartet und die Absorptionsänderung bei 37°C über 3 Minuten bestimmt und daraus das ΔE/min berechnet.

Die Aktivität wurde dann nach folgender Formel berechnet:

$$\text{Aktivität} = \frac{3{,}10}{\varepsilon \times 0{,}005 \times 1} \times \Delta E/\text{min} \times \frac{1}{\text{Faktor x}} \quad [\text{U/ml Probelösung}]$$

$\varepsilon = 18{,}2\ [1 \times \text{mmol}^{-1} \times \text{cm}^{-1}]$
Faktor x = Konzentrierungsfaktor nach Zellaufschluss

Analog wurde die Aktivität aus dem Mediumüberstand der Expressionskulturen bestimmt. Hier wurden ebenfalls die Reaktion mit 50 µl des Überstandes gestartet, jedoch wurden zusätzlich noch jeweils 0,5 mM $ZnCl_2$ zugegeben. Die Berechnung erfolgte dabei ohne Faktor x. Als Positivkontrolle wurden jeweils Überstände von Klonen, die die hochaktive Alkalische Phosphatase gemäß SEQ ID NO.: 3 exprimieren und als Negativkontrolle Klone, die nur mit Ausgangsvektor pPICZαA ohne Zielgen transformiert wurden, mitgeführt. Mit diesem Aktivitätstest wurden die Restaktivität der Mutanten wie folgt bestimmt:

| | |
|---|---|
| Einzelmutante Ser92Ala: | Reduktion der spezifischen Aktivität um Faktor ca. 5000 |
| Einzelmutante Gly322Phe: | Reduktion der spezifischen Aktivität um Faktor ca. 2500 |
| Doppelmutante His320Asn/Gly322Phe: | Reduktion der spezifischen Aktivität um Faktor ca. 10000 (nahe der Nachweisgrenze) |
| Triplemutante Ser92Ala/His320Asn/Gly322Phe: | Reduktion der spezifischen Aktivität um Faktor-10000 (nahe der Nachweisgrenze) |

## Beispiel 5:

### Nachweis der Expression der AP-Mutanten über Western-Blot:

10 µl unkonzentrierten Überstand bzw. Rohextrakt nach Zellaufschluß wurden auf ein 10%iges SDS-Gel (NovexPre-Cast Gel/Invitrogen) aufgetragen und die darin befindlichen Proteine durch Anlegen eines elektrischen Feldes der Grösse nach aufgetrennt. Die so aufgetrennten Proteine wurden auf Nitrocellulosemembran geblottet (Novex® Western Transfer Apparatus - XCell II ™ Blot Module/Invitrogen). Die Membran wurde nach dem Blotten zweimal mit 20 ml Reinstwasser für 5 Minuten gewaschen, anschließend in 10 ml Blocking Solution (Invitrogen) 30 Minuten geschüttelt. Die Membran wurde dann erneut zweimal mit 20 ml Reinstwasser für 5 Minuten gewaschen, anschließend in 10 ml mit Blocking Solution (Invitrogen), die diesmal Antikörper 1 (Anti-AP-rabbit/Rockland Inc. ist 1:5000 verdünnt aus einer Stammlösung 10 mg/ml) enthielt, 1 Stunde inkubiert. Darauf wurde viermal mit 20 ml einer Antikörperwaschlösung (Invitrogen) für jeweils 5 Minuten gewaschen und anschließend die Membran mit 10 ml der Sekundär-Antiköperlösung (enthält den Anti-Rabbit-Antikörper/Invitrogen) für 30 min inkubiert. Es folgte wiederum eine viermalige Waschung für jeweils 5 Minuten mit 20 ml einer Antikörperwaschlösung (Invitrogen) und eine dreimalige Waschung mit 20 ml Reinstwasser für jeweils 2 Minuten. Zur Anfärbung wurde die Membran dann 5 ml einer Färbelösung (chromogenes Substrat/ Invitrogen) für 1 - 60 Minuten inkubiert. Die Inkubationszeit richtet sich nach der Güte der Färbung. Nach optimaler Anfärbung wird die Membran dreimal mit 20 ml Reinstwasser für jeweils 2 Minuten gewaschen und anschließend die Membran bei Raumtemperatur getrocknet. Sämtliche Lösungen mit Ausnahme des Reinstwassers stammten von dem Western Breeze Chromogenic Immunodetection Kit von Invitrogen, alle Inkubationsschritte erfolgten bei Raumtemperatur. Die Durchführung erfolgte nach Angabe des Herstellers.

## Beispiel 6:

### Steigerung der Expressionsleistung durch Mehrfachtransformation

Die besten Klone aus den Expressionsversuchen wurden wiederum für die Elektroporation wie in Beispiel 2 beschrieben vorbereitet und wiederum mit 1 µg linearisierter Expressionsvektor-Vektor-DNA transformiert und der Transformationsansatz auf YPDS-Agarplatten (Invitrogen) mit 1000 bis 2000 µg/ml Zeocin® (Invitrogen) ausplattiert. Dadurch

wird der Selektionsdruck derart erhöht, daß nur Klone wachsen können, die mehrere Kopien des Expressionsvektors und somit auch mehrere Kopien jeweiligen Resistenzgens (hier Zeocin®) in das Genom integriert haben. Das Zeocin®-Resistenzprotein ist das Produkt des Bleomycingens von Streptoalloteichus hindusstanus (Chalmels, T. et al., Curr. Genet. 20 (1991), 309-314; Drocourt, D. et al., Nucleic Acid Research 18 (1990), 4009), das Zeocin® in einem stöchiometrischen Konzentrationsverhältnis bindet und somit der Zelle Resistenz gegenüber Zeocin® verleiht. Je höher die Konzentration an Zeocin® in den YPDS-Agarplatten, um so mehr Resistenzprotein muß die Zelle erzeugen, um das Zeocin® quantitativ zu binden und damit ein Wachstum zu ermöglichen. Dies ist u.a. möglich, wenn multiple Kopien des Resistenzgens in das Genom integriert werden. Klone wurden wie oben beschrieben auf Raster-MD-Platten überimpft und wiederum wie in Beispiel 2 beschrieben mittels PCR-Analyse auf korrekte Integration der jeweiligen Expressionskassette überprüft. Anschließend wurden diese Klone wiederum wie in Beispiel 4 und 5 beschrieben auf AP-Aktivität bzw. durch Westernblotanalyse getestet.

Beispiel 7:

Steigerung der Expressionsleistung durch Verwendung eines zweiten Selektionsdruckes

Eine Steigerung der Zeocin®-Konzentration über 2000 μg/ml führte nicht zu einer verbesserten Expressionsleistung der Alkalische Phosphatase-Mutanten. Um die Genkopienzahl der Gene gemäß SEQ ID NO's: 8-11, die für die Alkalische Phosphatase-Mutanten kodieren und für die Expression in Hefe codon-optimiert sind, in den Expressionsklonen weiter zu erhöhen, wurde die Integration zusätzlicher Expressionsvektoren in das Genom der aus Beispiel 6 hervorgegangenen Expressionsklone mit der höchsten Expressionsleistung über einen zweiten Selektionsdruck, bevorzugt G418 (Roche Diagnostics GmbH) selektioniert. Zu diesem Zweck wurde die gesamte Expressionskassette aus pNaAP31-1 , bestehend aus AOX 1-Promotor, Signalpeptid des α-Faktors aus Saccharomyces cerevisiae, codon-optimiertem Gen für die Alkalische Phosphatase-Mutanten gemäß SEQ ID NO's: 8-11 und AOX 1-Transkriptionsterminations-Region, mittels PCR durch entsprechend gewählte Primer isoliert und, wie unten beschrieben, in den Vektor pPIC9K kloniert, dessen Integration in das Genom von Pichia pastoris über G418 (Roche Diagnostics GmbH) selektioniert wird. Bei den hier verwendeten Primern 5'-Expr und 3'-Expr handelt es sich um die Sequenzen SEQ ID NO.: 20 und SEQ ID NO.: 21.

Der PCR-Ansatz wurde mittels Agarosegelelektrophorese analysiert, das Genfragment mit der erwarteten Größe isoliert (QIAquick Gel Extraction Kit/Qiagen), mit SacI und NotI (Roche Diagnostics GmbH) nachgeschnitten, anschließend wieder aus dem Agarosegel isoliert (QIAquick Gel Extraction Kit/Qiagen) und in ein ebenfalls mit SacI/NotI (Roche Diagnostics GmbH) linearisiertes und isoliertes Vektorfragment von pPIC9K ligiert. Somit war gewährleistet, daß die gesamte Expressionskassette aus den jeweiligen Expressionsvektoren identisch in pPIC9K vorlag. Das insertierte Fragment wurde mittels Restriktionsanalyse und Sequenzierung mit den flankierenden Regionen überprüft. Die so entstandenen Expressionsvektoren für Alkalische Phosphatase-Mutanten wurden pNaAP31-2(Ser92Ala), pNaAP43-2 (Gly322Phe), pNaAP51-2 (His320Asn/Gly322Phe) und pNaAP6-2 (Ser92Ala/His320Asn/Gly322Phe) genannt.

Die Klone mit der höchstens AP-Mutanten-Expressionsleistung aus der Mehrfachtransformation mit den Zeocin® als Selektionsmarker wurden wie in Beispiel 2 beschrieben für die Elektroporation vorbereitet und mit 1 μg mit SacI (Roche Diagnostics GmbH) linearisierten Vektorfragment von pNaAP31-2 und Derivate wie in Beispiel 2 beschrieben transformiert. Der Transformationsansatz wurde anschließend 1 bis 3 Tage bei 4°C in 1 M Sorbitol (ICN) aufbewahrt (zur Ausbildung der G418-Resistenz) und dann 100 bis 200 μl auf YPD-Platten (Invitrogen) mit 1, 2 bzw. 4 mg/ml G418 (Roche Diagnostics GmbH) ausplattiert und 3 bis 5 Tage bei 30°C inkubiert. Daraus resultierende Klone wurden wiederum wie beschrieben über den Aktivitätstest auf eine erhöhte Expression der eukaryontischen Alkalische Phosphatase-Mutanten untersucht.

## Patentansprüche

1. Mutanten der eukaryontischen Alkalischen Phosphatase, wobei

   - die zu mutierende Sequenz (Wildtyp) SEQ ID NO.: 2 ist, und
   - die Mutante eine um mindestens den Faktor 100 verringerte AP-Aktivität im Vergleich zum Wildtyp aufweist, und die Mutante eine oder mehrere der nachfolgend genannten Mutationen aufweist, wobei die Position der Mutation relativ zur Position in SEQ ID NO.: 2 definiert ist:

     Ser92: gegen Ala oder Gly
     His320: gegen Asn oder Phe
     Gly322: gegen Phe oder Lys

2. Mutanten der eukaryontischen Alkalischen Phosphatase gemäß Anspruch 1, wobei das Enzym nach der Wildtyp-Sequenz eine spezifische Aktivität von über 7000 U/mg aufweist.

3. Mutanten der eukaryontischen Alkalischen Phosphatase gemäß einem der Ansprüche 1 und 2, wobei die Mutante eine um mindestens den Faktor 1000 verringerte Aktivität im Vergleich zum Wildtyp aufweist.

4. Mutanten der eukaryontischen Alkalischen Phosphatase gemäß einem der Ansprüche 1 bis 3, wobei die Mutante eine um mindestens den Faktor 10000 verringerte Aktivität im Vergleich zum Wildtyp aufweist.

5. Mutanten der eukaryontischen Alkalischen Phosphatase gemäß einem der Ansprüche 1 bis 4, wobei diese Mutante eine Aminosäuresequenz aufweist, welche ausgewählt ist aus den SEQ ID NO's: 4-7.

6. Rekombinante DNA kodierend eine Mutante der eukaryontischen Alkalischen Phosphatase gemäß einem der Ansprüche 1-5.

7. Rekombinante DNA kodierend eine Mutante der eukaryontischen Alkalischen Phosphatase wobei diese Mutante eine Nukleinsäuresequenz aufweist, welche ausgewählt ist aus den SEQ ID NO's: 8-11.

8. Vektor enthaltend eine Nukleinsäuresequenz , welche ausgewählt ist aus den SEQ ID NO's: 8-11.

9. Vektor gemäß Anspruch 8, der einem Vektor entspricht, welcher ausgewählt ist aus den folgenden Vektoren: pPICZαA, B, C;pPICZ, pPICZ-E, pPICZα-E; pPIC6, pPIC6αA, B, C; pGAPZ, pGAPZαA, B, C; pPIC9; pPIC9K, pPIC3.5, pPIC3.5K, pAO815, pMET, pMETαA, B, C;pYES-Dest52, pYES2.1/V5-His-TOPO, pYC2-E, pYES2.1-E; Yes-Vektoren, pTEF1/Zeo, pTEF1/Bsd, pNMT-TOPO.

10. Wirtstamm transformiert mit einem Vektor gemäß Anspruch 8 oder 9.

11. Wirtsstamm gemäß Anspruch 10, wobei als Wirtstamm Pichia pastoris verwendet wird.

12. Verfahren zur Herstellung einer Mutante der eukaryontischen Alkalischen Phosphatase gemäß einem der Ansprüche 1-5 in Hefezellen, umfassend die Schritte: a) Klonierung einer Gensequenz gemäß Anspruch 6 oder 7 in unterschiedlichen Vektoren, b) Transformation der Hefe, c) Expression der AP-Mutante und d) Reinigung der Alkalischen Phosphatase, **dadurch gekennzeichnet, daß**

    - ein erster Vektor ein Resistenzgen gegen einen ersten Selektionsmarker aufweist,
    - Transformanten, die das Resistenzgen und die gewünschte Gensequenz in das Genom integriert haben, durch Wachstum auf Nährmedium mit einer geringen Konzentration an erstem Selektionsmarker selektioniert werden,
    - die Genkopienzahl durch Mehrfachtransformation erhöht wird, wobei Mehrfachtransformanten durch Wachstum auf Nährmedium mit erhöhtem Selektionsdruck selektioniert werden,
    - ein zweiter Vektor, welcher ein Resistenzgen gegen einen zweiten Selektionsmarker aufweist, zugegeben wird,
    - die Genkopienzahl durch Mehrfachtransformation mit dem zweiten Vektor erhöht wird, wobei Mehrfachtransformanten durch Wachstum auf Nährmedium mit erhöhtem Selektionsdruck selektioniert werden, und
    solche Klone selektioniert werden, die mehrere Kopien der Gensequenz und der Selektionsmarker-Resistenzgene stabil in das Genom integriert haben.

**13.** Verfahren gemäß Anspruch 12, wobei methylotrophe Hefezellen verwendet werden.

**14.** Verfahren gemäß Anspruch 13, wobei als Hefezelle Pichia pastoris verwendet wird.

**15.** Verfahren gemäß einem der Ansprüche 12-14, wobei als Vektor einer verwendet, der einem Vektor entspricht, welcher ausgewählt ist aus den folgenden Vektoren: pPICZαA, B, C;pPICZ, pPICZ-E, pPICZα-E; pPIC6, pPIC6αA, B, C; pGAPZ, pGAPZαA, B, C; pPIC9; pPIC9K, pPIC3.5, pPIC3.5K, pAO815.

## Claims

**1.** Mutants of eukaryotic alkaline phosphatase, wherein

- the sequence (wild type) to be mutated is SEQ ID NO.: 2 and
- the mutant has an at least 100-fold reduced AP activity compared to the wild type and

the mutant has one or more of the mutations mentioned below, wherein the position of the mutation is defined relative to the position in SEQ ID NO.: 2:

Ser92: by Ala or Gly
His320: by Asn or Phe
Gly322: by Phe or Lys.

**2.** Mutants of eukaryotic alkaline phosphatase according to claim 1, wherein the enzyme having the wild type sequence has a specific activity of more than 7000 U/mg.

**3.** Mutants of eukaryotic alkaline phosphatase according to one of the claims 1 and 2, wherein the mutant has an at least 1000-fold reduced activity compared to the wild type.

**4.** Mutants of eukaryotic alkaline phosphatase according to one of the claims 1 to 3, wherein the mutant has an at least 10000-fold reduced activity compared to the wild type.

**5.** Mutants of eukaryotic alkaline phosphatase according to one of the claims 1 to 4, wherein this mutant has an amino acid sequence which is selected from the SEQ ID NOs: 4-7.

**6.** Recombinant DNA coding for a mutant of eukaryotic alkaline phosphatase according to one of the claims 1-5.

**7.** Recombinant DNA coding for a mutant of eukaryotic alkaline phosphatase, wherein this mutant has a nucleic acid sequence which is selected from SEQ ID NOs.: 8-11.

**8.** Vector containing a nucleic acid sequence which is selected from the sequences SEQ ID NOs: 8-11.

**9.** Vector according to claim 8, which corresponds to a vector which is selected from the following vectors: pPICZαA, B, C; pPICZ, pPICZ-E, pPICZα-E; pPIC6, pPIC6αA, B, C; pGAPZ, pGAPZαA, B, C; pPIC9; pPIC9K, pPIC3.5, pPIC3.5K, pAO815, pMET, pMETαA, B, C; pYES-Dest52, pYES2.1/V5-His-TOPO, pYC2-E, pYES2.1-E; Yes-vectors, pTEF1/Zeo, pTEF1/Bsd, pNMT-TOPO.

**10.** Host strain transformed with a vector according to claim 8 or 9.

**11.** Host strain according to claim 10, wherein Pichia pastoris is used as the host strain.

**12.** Method for producing a mutant of eukaryotic alkaline phosphatase according to one of the claims 1-5 in yeast cells, comprising the steps: a) cloning a gene sequence according to claim 6 or 7 in various vectors, b) transforming the yeast, c) expressing the AP mutant and d) purifying the alkaline phosphatase,
**characterized in that**

- a first vector has a resistance gene against a first selection marker,
- transformants which have integrated the resistance gene and the desired gene sequence into the genome are selected by growth on a nutrient medium containing a low concentration of the first selection marker,

- the gene copy number is increased by multiple transformation and multiple transformants are selected by growth on a nutrient medium under increased selection pressure,
- a second vector which has a resistance gene against a second selection marker is added,
- the gene copy number is increased by multiple transformation with the second vector and multiple transformants are selected by growth on a nutrient medium under increased selection pressure, and those clones are selected which have stably integrated several copies of the gene sequence and of the selection marker resistance genes into the genome.

**13.** Method according to claim 12, wherein methylotrophic yeast cells are used.

**14.** Method according to claim 13, wherein Pichia pastoris is used as the yeast cell.

**15.** Method according to one of the claims 12-14, wherein a vector is used which corresponds to a vector that is selected from the following vectors: pPICZαA, B, C; pPICZ, pPICZ-E, pPICZα-E; pPIC6, pPIC6αA, B, C; pGAPZ, pGAPZαA, B, C; pPIC9; pPIC9K, pPIC3.5, pPIC3.5K, pAO815.

## Revendications

**1.** Mutants de la phosphatase alcaline eucaryote, où

- la séquence à muter (de type sauvage) est la séquence SEQ ID NO. : 2, et
- le mutant présente une activité d'AP diminuée d'au moins un facteur 100 par rapport au type sauvage, et

le mutant présente une ou plusieurs des mutations mentionnées ci-dessous, la position de la mutation étant définie par rapport à la position dans SEQ ID NO. : 2:

Ser92 : remplacé par Ala ou Gly
His320 : remplacé par Asn ou Phe
Gly322 : remplacé par Phe ou Lys.

**2.** Mutants de la phosphatase alcaline eucaryote selon la revendication 1, où l'enzyme selon la séquence de type sauvage présente une activité spécifique supérieure à 7000 U/mg.

**3.** Mutants de la phosphatase alcaline eucaryote selon l'une quelconque des revendications 1 et 2, le mutant présentant une activité diminuée d'au moins un facteur 1000 par rapport au type sauvage.

**4.** Mutants de la phosphatase alcaline eucaryote selon l'une quelconque des revendications 1 à 3, le mutant présentant une activité diminuée d'au moins un facteur 10 000 par rapport au type sauvage.

**5.** Mutants de la phosphatase alcaline eucaryote selon l'une quelconque des revendications 1 à 4, ce mutant présentant une séquence d'acides aminés choisie parmi les SEQ ID NO : 4-7.

**6.** ADN recombinant codant pour un mutant de la phosphatase alcaline eucaryote selon l'une quelconque des revendications 1-5.

**7.** ADN recombinant codant pour un mutant de la phosphatase alcaline eucaryote, ce mutant présentant une séquence d'acides nucléiques choisie parmi les SEQ ID NO : 8-11.

**8.** Vecteur contenant une séquence d'acides nucléiques qui est choisie parmi les SEQ ID NO : 8-11.

**9.** Vecteur selon la revendication 8, qui correspond à un vecteur choisi parmi les vecteurs suivants : pPICZαA, B, C ; pPICZ, pPICZ-E, pPICZα-E ; pPIC6, pPIC6αA, B, C; pGAPZ, pGAPZαA, B, C; pPIC9 ; pPIC9K, pPIC3.5, pPIC3.5K, pAO815, pMET, pMETαA, B, C; pYES-Dest52, pYES2,1/V5-His-TOPO, pYC2-E, pYES2.1-E ; vecteurs Yes, pTEF1/Zeo, pTEF1/Bsd, pNMT-TOPO.

**10.** Souche hôte transformée avec un vecteur selon la revendication 8 ou 9.

**11.** Souche hôte selon la revendication 10, où on utilise comme souche hôte la souche Pichia pastoris.

**12.** Procédé pour la préparation d'un mutant de la phosphatase alcaline eucaryote selon l'une quelconque des revendications 1-5 dans des cellules de levure, comprenant les étapes : a) clonage d'une séquence génique selon la revendication 6 ou 7 dans différents vecteurs, b) transformation de la levure, c) expression du mutant d'AP et d) purification de la phosphatase alcaline,
**caractérisé en ce que**

- un premier vecteur présente un gène de résistance à un premier marqueur de sélection,
- des transformants qui ont intégré le gène de résistance et la séquence génique souhaitée dans le génome sont sélectionnés par croissance sur un milieu nutritif à une faible concentration par rapport au premier marqueur de sélection,
- le nombre de copies géniques est augmenté par une transformation multiple, les transformants multiples étant sélectionnés par croissance sur un milieu nutritif avec une pression de sélection élevée,
- un deuxième vecteur, qui présente un gène de résistance à un deuxième marqueur de sélection est ajouté,
- le nombre de copies géniques est augmenté par une transformation multiple avec le deuxième vecteur, les transformants multiples étant sélectionnés par croissance sur un milieu nutritif avec une pression de sélection élevée, et

les clones qui ont intégré de manière stable dans le génome plusieurs copies de la séquence génique et des gènes de résistance au marqueur de sélection sont sélectionnés.

**13.** Procédé selon la revendication 12, où on utilise des cellules de levure méthylotrophes.

**14.** Procédé selon la revendication 13, où on utilise comme cellule de levure la cellule Pichia pastoris.

**15.** Procédé selon l'une quelconque des revendications 12-14, où on utilise comme vecteur un vecteur qui correspond à un vecteur choisi parmi les vecteurs suivants : pPICZαA , B, C ; pPICZ, pPICZ-E, pPICZα-E ; pPIC6, pPIC6αA, B, C; pGAPZ, pGAPZαA, B, C; pPIC9 ; pPIC9K, pPIC3.5, pPIC3.5K, pA0815.

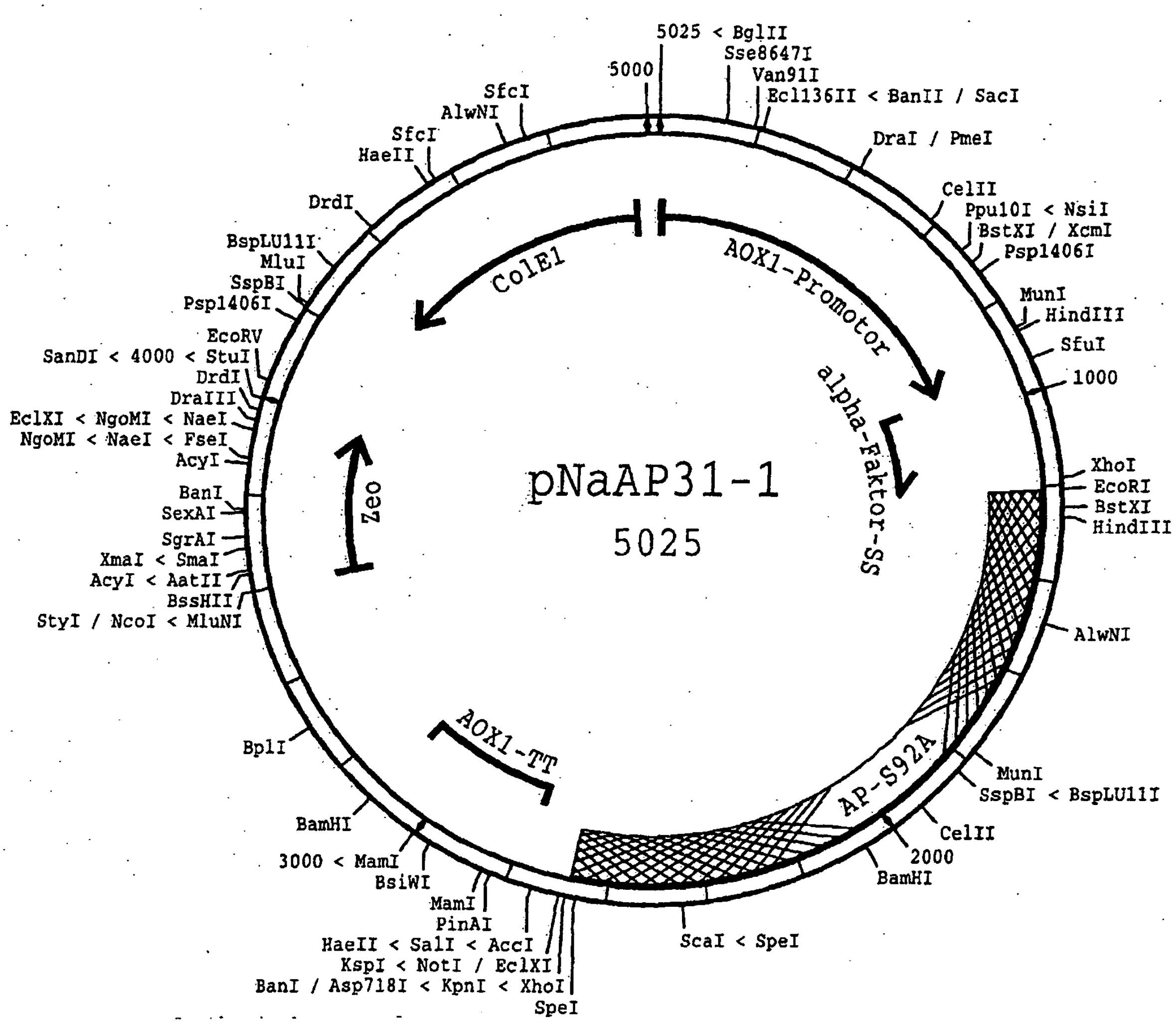


Fig. 1 Plasmidkarte von dem Expressionsvektor pNaAP31-1, der das Gen für die Alkalische Phosphatase-Einzelmutante Ser92Ala besitzt. pNaAP31-1 ist beispielhaft dargestellt für die weiteren Expressionsvektoren pNaAP43-1, pNaAP51-1 und pNaAP6-1

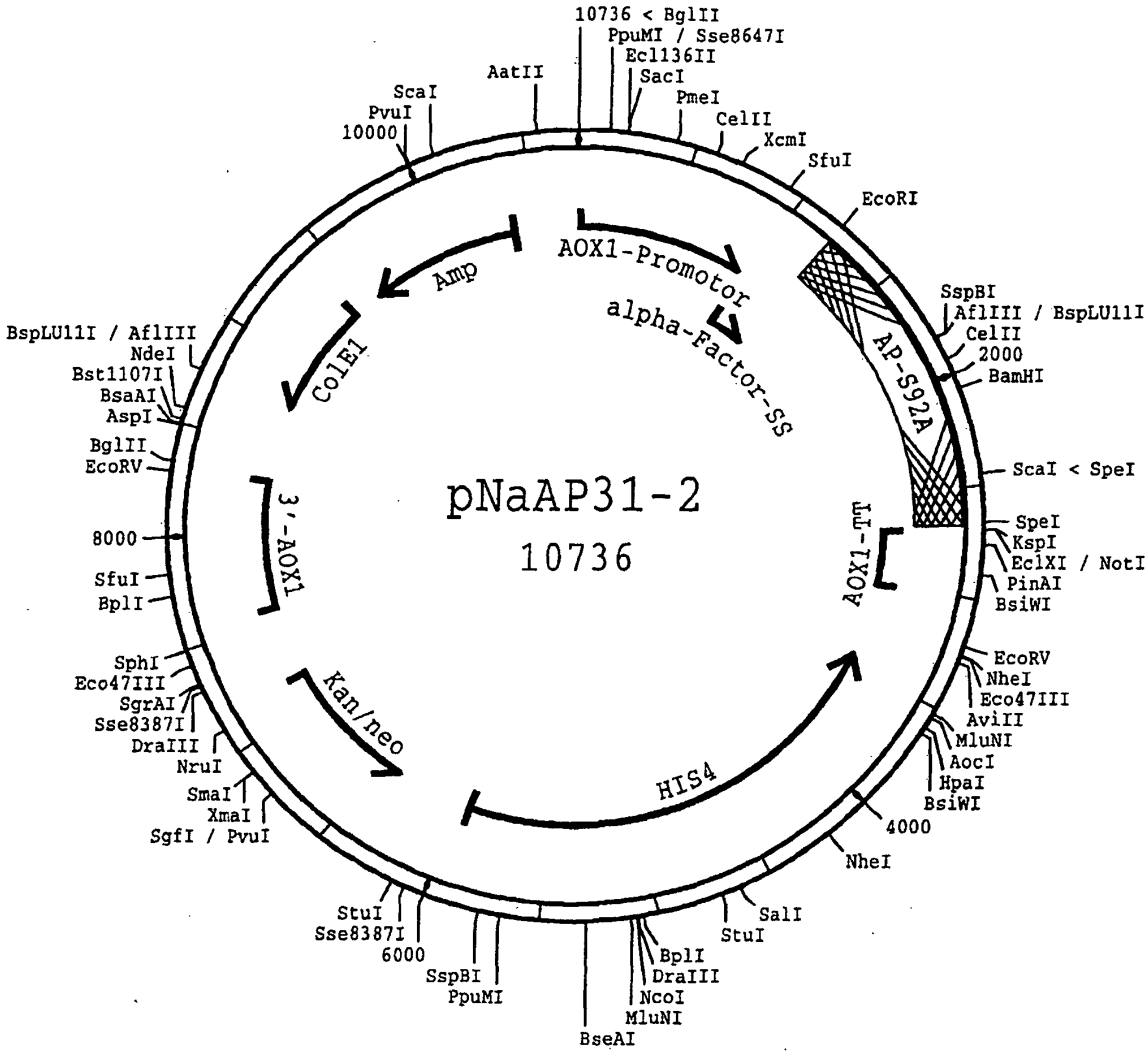


Fig. 2 Plasmidkarte von dem Expressionsvektor pNaAP31-2, der das Gen für die Alkalische Phosphatase-Einzelmutante Ser92Ala besitzt. pNaAP31-1 ist beispielhaft dargestellt für die weiteren Expressionsvektoren pNaAP43-2, pNaAP51-2 und pNaAP6-2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0955369 A
- WO 9318139 A


### In der Beschreibung aufgeführte Nicht-Patentliteratur


- **McComb et al.** Alkaline Phosphatases. Plenum Press, 1979
- **Millàn.** *Anticancer Res.,* 1988, vol. 8, 995-1004
- **Harris.** *Clin. Chim. Acta,* 1989, vol. 186, 133-150
- **Millan.** *Anticancer Res.,* 1988, vol. 8, 995-1004
- **Manes et al.** *J. Biol. Chem,* 1998, vol. 273 (36), 23353-23360
- **Weissig et al.** *Biochem J,* 1993, vol. 260, 503-508
- **Berger et al.** *Biochemistry,* 1987, vol. 84, 4885-4889
- **Davis et al.** *Biotechnology,* 1992, vol. 10, 1148-1150
- **Beck ; Burtscher.** *Protein Expression and Purification,* 1994, vol. 5, 192-197
- **Ma ; Kantrowitz.** *J. Biol. Chem,* 1994, vol. 16, 31614-31619
- **Ma et al.** *Protein Science,* 1995, vol. 4, 1498-1506
- **Kimura ; Kikuta.** *JBIC,* 2000, vol. 5, 139-155
- **Stec et al.** *JMB,* 2000, vol. 299, 1303-1311
- **Cregg, J.M. et al.** *Mol. Cell. Biol.,* 1985, vol. 5, 3376-3385
- Protein Purification. Springer-Verlag, 1982
- **Chalmels, T. et al.** *Curr. Genet.,* 1991, vol. 20, 309-314
- **Drocourt, D. et al.** *Nucleic Acid Research,* 1990, vol. 18, 4009

SEQUENZPROTOKOLL

<110> Roche Diagnostics GmbH

<120> Erzeugung von schwach aktiven oder inaktiven Mutanten von Alkalischer Phosphatase und deren Expression in Hefe

<130> W 21227 DE

<160> 21

<170> PatentIn version 3.1

<210> 1
<211> 1464
<212> DNA
<213> Bovine

<220>
<221> CDS
<222> (1)..(1464)
<223>

<400> 1

```
ctc atc cca gct gag gag gaa aac ccc gcc ttc tgg aac cgc cag gca      48
Leu Ile Pro Ala Glu Glu Glu Asn Pro Ala Phe Trp Asn Arg Gln Ala
1               5                   10                  15

gcc cag gcc ctt gat gta gcc aag aag ttg cag ccg atc cag aca gct      96
Ala Gln Ala Leu Asp Val Ala Lys Lys Leu Gln Pro Ile Gln Thr Ala
            20                  25                  30

gcc aag aat gtc atc ctc ttc ttg ggg gat ggg atg ggg gtg cct acg      144
Ala Lys Asn Val Ile Leu Phe Leu Gly Asp Gly Met Gly Val Pro Thr
        35                  40                  45

gtg aca gcc act cgg atc cta aag ggg cag atg aat ggc aaa ctg gga      192
Val Thr Ala Thr Arg Ile Leu Lys Gly Gln Met Asn Gly Lys Leu Gly
    50                  55                  60

cct gag aca ccc ctg gcc atg gac cag ttc cca tac gtg gct ctg tcc      240
Pro Glu Thr Pro Leu Ala Met Asp Gln Phe Pro Tyr Val Ala Leu Ser
65                  70                  75                  80

aag aca tac aac gtg gac aga cag gtg cca gac agc gca ggc act gcc      288
Lys Thr Tyr Asn Val Asp Arg Gln Val Pro Asp Ser Ala Gly Thr Ala
                85                  90                  95
```

```
act gcc tac ctg tgt ggg gtc aag ggc aac tac aga acc atc ggt gta      336
Thr Ala Tyr Leu Cys Gly Val Lys Gly Asn Tyr Arg Thr Ile Gly Val
            100                 105                 110

agt gca gcc gcc cgc tac aat cag tgc aac acg aca cgt ggg aat gag      384
Ser Ala Ala Ala Arg Tyr Asn Gln Cys Asn Thr Thr Arg Gly Asn Glu
        115                 120                 125

gtc acg tct gtg atc aac cgg gcc aag aaa gca ggg aag gcc gtg gga      432
Val Thr Ser Val Ile Asn Arg Ala Lys Lys Ala Gly Lys Ala Val Gly
    130                 135                 140

gtg gtg acc acc acc agg gtg cag cat gcc tcc cca gcc ggg gcc tac      480
Val Val Thr Thr Thr Arg Val Gln His Ala Ser Pro Ala Gly Ala Tyr
145                 150                 155                 160

gcg cac acg gtg aac cga aac tgg tac tca gac gcc gac ctg cct gct      528
Ala His Thr Val Asn Arg Asn Trp Tyr Ser Asp Ala Asp Leu Pro Ala
                165                 170                 175

gat gca cag aag aat ggc tgc cag gac atc gcc gca cag ctg gtc tac      576
Asp Ala Gln Lys Asn Gly Cys Gln Asp Ile Ala Ala Gln Leu Val Tyr
            180                 185                 190

aac atg gat att gac gtg atc ctg ggt gga ggc cga atg tac atg ttt      624
Asn Met Asp Ile Asp Val Ile Leu Gly Gly Gly Arg Met Tyr Met Phe
        195                 200                 205

cct gag ggg acc cca gac cct gaa tac cca gat gat gcc agt gtg aat      672
Pro Glu Gly Thr Pro Asp Pro Glu Tyr Pro Asp Asp Ala Ser Val Asn
    210                 215                 220

gga gtc cgg aag gac aag cag aac ctg gtg cag gaa tgg cag gcc aag      720
Gly Val Arg Lys Asp Lys Gln Asn Leu Val Gln Glu Trp Gln Ala Lys
225                 230                 235                 240

cac cag gga gcc cag tat gtg tgg aac cgc act gcg ctc ctt cag gcg      768
His Gln Gly Ala Gln Tyr Val Trp Asn Arg Thr Ala Leu Leu Gln Ala
                245                 250                 255

gcc gat gac tcc agt gta aca cac ctc atg ggc ctc ttt gag ccg gca      816
Ala Asp Asp Ser Ser Val Thr His Leu Met Gly Leu Phe Glu Pro Ala
            260                 265                 270

gac atg aag tat aat gtt cag caa gac cac acc aag gac ccg acc ctg      864
Asp Met Lys Tyr Asn Val Gln Gln Asp His Thr Lys Asp Pro Thr Leu
        275                 280                 285

gcg gag atg acg gag gcg gcc ctg caa gtg ctg agc agg aac ccc cgg      912
Ala Glu Met Thr Glu Ala Ala Leu Gln Val Leu Ser Arg Asn Pro Arg
    290                 295                 300

ggc ttc tac ctc ttc gtg gag gga ggc cgc att gac cac ggt cac cat      960
Gly Phe Tyr Leu Phe Val Glu Gly Gly Arg Ile Asp His Gly His His
305                 310                 315                 320
```

```
gac ggc aaa gct tat atg gca ctg act gag gcg atc atg ttt gac aat      1008
Asp Gly Lys Ala Tyr Met Ala Leu Thr Glu Ala Ile Met Phe Asp Asn
                325                 330                 335

gcc atc gcc aag gct aac gag ctc act agc gaa ctg gac acg ctg atc      1056
Ala Ile Ala Lys Ala Asn Glu Leu Thr Ser Glu Leu Asp Thr Leu Ile
            340                 345                 350

ctt gtc act gca gac cac tcc cat gtc ttc tct ttt ggt ggc tac aca      1104
Leu Val Thr Ala Asp His Ser His Val Phe Ser Phe Gly Gly Tyr Thr
        355                 360                 365

ctg cgt ggg acc tcc att ttc ggt ctg gcc ccc ggc aag gcc tta gac      1152
Leu Arg Gly Thr Ser Ile Phe Gly Leu Ala Pro Gly Lys Ala Leu Asp
    370                 375                 380

agc aag tcc tac acc tcc atc ctc tat ggc aat ggc cca ggc tat gcg      1200
Ser Lys Ser Tyr Thr Ser Ile Leu Tyr Gly Asn Gly Pro Gly Tyr Ala
385                 390                 395                 400

ctt ggc ggg ggc tcg agg ccc gat gtt aat ggc agc aca agc gag gaa      1248
Leu Gly Gly Gly Ser Arg Pro Asp Val Asn Gly Ser Thr Ser Glu Glu
                405                 410                 415

ccc tca tac cgg cag cag gcg gcc gtg ccc ctg gct agc gag acc cac      1296
Pro Ser Tyr Arg Gln Gln Ala Ala Val Pro Leu Ala Ser Glu Thr His
            420                 425                 430

ggg ggc gaa gac gtg gcg gtg ttc gcg cga ggc ccg cag gcg cac ctg      1344
Gly Gly Glu Asp Val Ala Val Phe Ala Arg Gly Pro Gln Ala His Leu
        435                 440                 445

gtg cac ggc gtg cag gag gag acc ttc gtg gcg cac atc atg gcc ttt      1392
Val His Gly Val Gln Glu Glu Thr Phe Val Ala His Ile Met Ala Phe
    450                 455                 460

gcg ggc tgc gtg gag ccc tac acc gac tgc aat ctg cca gcc ccc gcc      1440
Ala Gly Cys Val Glu Pro Tyr Thr Asp Cys Asn Leu Pro Ala Pro Ala
465                 470                 475                 480

acc gcc acc agc atc ccc gac tag                                      1464
Thr Ala Thr Ser Ile Pro Asp
                485
```

<210> 2
<211> 487
<212> PRT
<213> Bovine

<400> 2

Leu Ile Pro Ala Glu Glu Glu Asn Pro Ala Phe Trp Asn Arg Gln Ala
1               5                   10                  15

Ala Gln Ala Leu Asp Val Ala Lys Lys Leu Gln Pro Ile Gln Thr Ala
            20                  25                  30

Ala Lys Asn Val Ile Leu Phe Leu Gly Asp Gly Met Gly Val Pro Thr
        35                  40                  45

Val Thr Ala Thr Arg Ile Leu Lys Gly Gln Met Asn Gly Lys Leu Gly
    50                  55                  60

Pro Glu Thr Pro Leu Ala Met Asp Gln Phe Pro Tyr Val Ala Leu Ser
65                  70                  75                  80

Lys Thr Tyr Asn Val Asp Arg Gln Val Pro Asp Ser Ala Gly Thr Ala
                85                  90                  95

Thr Ala Tyr Leu Cys Gly Val Lys Gly Asn Tyr Arg Thr Ile Gly Val
            100                 105                 110

Ser Ala Ala Ala Arg Tyr Asn Gln Cys Asn Thr Thr Arg Gly Asn Glu
        115                 120                 125

Val Thr Ser Val Ile Asn Arg Ala Lys Lys Ala Gly Lys Ala Val Gly
    130                 135                 140

Val Val Thr Thr Thr Arg Val Gln His Ala Ser Pro Ala Gly Ala Tyr
145                 150                 155                 160

Ala His Thr Val Asn Arg Asn Trp Tyr Ser Asp Ala Asp Leu Pro Ala
                165                 170                 175

Asp Ala Gln Lys Asn Gly Cys Gln Asp Ile Ala Ala Gln Leu Val Tyr
            180                 185                 190

Asn Met Asp Ile Asp Val Ile Leu Gly Gly Gly Arg Met Tyr Met Phe
        195                 200                 205

Pro Glu Gly Thr Pro Asp Pro Glu Tyr Pro Asp Asp Ala Ser Val Asn
    210                 215                 220

Gly Val Arg Lys Asp Lys Gln Asn Leu Val Gln Glu Trp Gln Ala Lys
225                 230                 235                 240

His Gln Gly Ala Gln Tyr Val Trp Asn Arg Thr Ala Leu Leu Gln Ala
                245                 250                 255

Ala Asp Asp Ser Ser Val Thr His Leu Met Gly Leu Phe Glu Pro Ala
            260                 265                 270

Asp Met Lys Tyr Asn Val Gln Gln Asp His Thr Lys Asp Pro Thr Leu
        275                 280                 285

Ala Glu Met Thr Glu Ala Ala Leu Gln Val Leu Ser Arg Asn Pro Arg
290 295 300

Gly Phe Tyr Leu Phe Val Glu Gly Gly Arg Ile Asp His Gly His His
305 310 315 320

Asp Gly Lys Ala Tyr Met Ala Leu Thr Glu Ala Ile Met Phe Asp Asn
325 330 335

Ala Ile Ala Lys Ala Asn Glu Leu Thr Ser Glu Leu Asp Thr Leu Ile
340 345 350

Leu Val Thr Ala Asp His Ser His Val Phe Ser Phe Gly Gly Tyr Thr
355 360 365

Leu Arg Gly Thr Ser Ile Phe Gly Leu Ala Pro Gly Lys Ala Leu Asp
370 375 380

Ser Lys Ser Tyr Thr Ser Ile Leu Tyr Gly Asn Gly Pro Gly Tyr Ala
385 390 395 400

Leu Gly Gly Gly Ser Arg Pro Asp Val Asn Gly Ser Thr Ser Glu Glu
405 410 415

Pro Ser Tyr Arg Gln Gln Ala Ala Val Pro Leu Ala Ser Glu Thr His
420 425 430

Gly Gly Glu Asp Val Ala Val Phe Ala Arg Gly Pro Gln Ala His Leu
435 440 445

Val His Gly Val Gln Glu Glu Thr Phe Val Ala His Ile Met Ala Phe
450 455 460

Ala Gly Cys Val Glu Pro Tyr Thr Asp Cys Asn Leu Pro Ala Pro Ala
465 470 475 480

Thr Ala Thr Ser Ile Pro Asp
485

<210> 3
<211> 1476
<212> DNA
<213> Nucleic acid

<400> 3

gaattcttga ttccagctga agaagaaaat ccagcttttt ggaatagaca agctgctcaa 60

gctttggatg ttgctaagaa gttgcaacca attcaaactg ctgctaagaa tgttattttg 120

tttttgggtg atggtatggg tgttccaact gttactgcta ctagaatttt gaagggtcaa 180

```
atgaatggta agttgggtcc agaaactcca ttggctatgg atcaatttcc atacgttgct    240
ttgtctaaga cttacaatgt tgatagacaa gttccagatt ctgctggtac tgctactgct    300
tacttgtgtg gtgttaaggg taattacaga actattggtg tttctgctgc tgctagatac    360
aatcaatgta atactactag aggtaatgaa gttacttctg ttattaatag agctaagaag    420
gctggtaagg ctgttggtgt tgttactact actagagttc aacatgcttc tccagctggt    480
gcttacgctc atactgttaa tagaaattgg tactctgatg ctgatttgcc agctgatgct    540
caaaagaatg gttgtcaaga tattgctgct caattggttt acaatatgga tattgatgtt    600
attttgggtg gtggtagaat gtacatgttt ccagaaggta ctccagatcc agaataccca    660
gatgatgctt ctgttaatgg tgttagaaag gataagcaaa atttggttca agaatggcaa    720
gctaagcatc aaggtgctca atatgtttgg aatagaactg ctttgttgca agctgctgat    780
gattctagtg ttactcattt gatgggtttg tttgaaccag ctgatatgaa gtataatgtt    840
caacaagatc atactaagga tccaactttg gctgaaatga ctgaagctgc tttgcaagtt    900
ttgtctagaa atccaagagg tttttacttg tttgttgaag gtggtagaat tgatcatggt    960
catcatgatg gtaaggctta tatggctttg actgaagcta ttatgtttga taatgctatt   1020
gctaaggcta atgaattgac ttctgaattg gatactttga ttttggttac tgctgatcat   1080
agtcatgttt tttcttttgg tggttacact ttgagaggta cttctatttt tggtttggct   1140
ccaggtaagg ctttggatag taagtcttac acttctattt tgtatggtaa tggtccaggt   1200
tatgctttgg gtggtggttc tagaccagat gttaatggta gtactagtga agaaccatct   1260
tacagacaac aagctgctgt tccattggct agtgaaactc atggtggtga agatgttgct   1320
gtttttgcta gaggtccaca agctcatttg gttcatggtg ttcaagaaga aacttttgtt   1380
gctcatatta tggcttttgc tggttgtgtt gaaccataca ctgattgtaa tttgccagct   1440
ccagctactg ctactagtat tccagattaa ggtacc                              1476
```

<210> 4
<211> 487
<212> PRT
<213> Bovine/mutated

<400> 4

```
Leu Ile Pro Ala Glu Glu Glu Asn Pro Ala Phe Trp Asn Arg Gln Ala
1               5                   10                  15

Ala Gln Ala Leu Asp Val Ala Lys Lys Leu Gln Pro Ile Gln Thr Ala
            20                  25                  30

Ala Lys Asn Val Ile Leu Phe Leu Gly Asp Gly Met Gly Val Pro Thr
        35                  40                  45

Val Thr Ala Thr Arg Ile Leu Lys Gly Gln Met Asn Gly Lys Leu Gly
    50                  55                  60

Pro Glu Thr Pro Leu Ala Met Asp Gln Phe Pro Tyr Val Ala Leu Ser
65                  70                  75                  80

Lys Thr Tyr Asn Val Asp Arg Gln Val Pro Asp Ala Ala Gly Thr Ala
                85                  90                  95

Thr Ala Tyr Leu Cys Gly Val Lys Gly Asn Tyr Arg Thr Ile Gly Val
            100                 105                 110

Ser Ala Ala Ala Arg Tyr Asn Gln Cys Asn Thr Thr Arg Gly Asn Glu
        115                 120                 125

Val Thr Ser Val Ile Asn Arg Ala Lys Lys Ala Gly Lys Ala Val Gly
    130                 135                 140

Val Val Thr Thr Thr Arg Val Gln His Ala Ser Pro Ala Gly Ala Tyr
145                 150                 155                 160

Ala His Thr Val Asn Arg Asn Trp Tyr Ser Asp Ala Asp Leu Pro Ala
                165                 170                 175

Asp Ala Gln Lys Asn Gly Cys Gln Asp Ile Ala Ala Gln Leu Val Tyr
            180                 185                 190

Asn Met Asp Ile Asp Val Ile Leu Gly Gly Gly Arg Met Tyr Met Phe
        195                 200                 205

Pro Glu Gly Thr Pro Asp Pro Glu Tyr Pro Asp Asp Ala Ser Val Asn
    210                 215                 220

Gly Val Arg Lys Asp Lys Gln Asn Leu Val Gln Glu Trp Gln Ala Lys
225                 230                 235                 240

His Gln Gly Ala Gln Tyr Val Trp Asn Arg Thr Ala Leu Leu Gln Ala
                245                 250                 255

Ala Asp Asp Ser Ser Val Thr His Leu Met Gly Leu Phe Glu Pro Ala
            260                 265                 270

Asp Met Lys Tyr Asn Val Gln Gln Asp His Thr Lys Asp Pro Thr Leu
        275                 280                 285
```

Ala Glu Met Thr Glu Ala Ala Leu Gln Val Leu Ser Arg Asn Pro Arg
290 295 300

Gly Phe Tyr Leu Phe Val Glu Gly Gly Arg Ile Asp His Gly His His
305 310 315 320

Asp Gly Lys Ala Tyr Met Ala Leu Thr Glu Ala Ile Met Phe Asp Asn
325 330 335

Ala Ile Ala Lys Ala Asn Glu Leu Thr Ser Glu Leu Asp Thr Leu Ile
340 345 350

Leu Val Thr Ala Asp His Ser His Val Phe Ser Phe Gly Gly Tyr Thr
355 360 365

Leu Arg Gly Thr Ser Ile Phe Gly Leu Ala Pro Gly Lys Ala Leu Asp
370 375 380

Ser Lys Ser Tyr Thr Ser Ile Leu Tyr Gly Asn Gly Pro Gly Tyr Ala
385 390 395 400

Leu Gly Gly Gly Ser Arg Pro Asp Val Asn Gly Ser Thr Ser Glu Glu
405 410 415

Pro Ser Tyr Arg Gln Gln Ala Ala Val Pro Leu Ala Ser Glu Thr His
420 425 430

Gly Gly Glu Asp Val Ala Val Phe Ala Arg Gly Pro Gln Ala His Leu
435 440 445

Val His Gly Val Gln Glu Glu Thr Phe Val Ala His Ile Met Ala Phe
450 455 460

Ala Gly Cys Val Glu Pro Tyr Thr Asp Cys Asn Leu Pro Ala Pro Ala
465 470 475 480

Thr Ala Thr Ser Ile Pro Asp
485

<210> 5
<211> 487
<212> PRT
<213> Bovine/mutated

<400> 5

Leu Ile Pro Ala Glu Glu Glu Asn Pro Ala Phe Trp Asn Arg Gln Ala
1 5 10 15

Ala Gln Ala Leu Asp Val Ala Lys Lys Leu Gln Pro Ile Gln Thr Ala
20 25 30

```
Ala Lys Asn Val Ile Leu Phe Leu Gly Asp Gly Met Gly Val Pro Thr
        35                  40                  45

Val Thr Ala Thr Arg Ile Leu Lys Gly Gln Met Asn Gly Lys Leu Gly
    50                  55                  60

Pro Glu Thr Pro Leu Ala Met Asp Gln Phe Pro Tyr Val Ala Leu Ser
65                  70                  75                  80

Lys Thr Tyr Asn Val Asp Arg Gln Val Pro Asp Ser Ala Gly Thr Ala
                85                  90                  95

Thr Ala Tyr Leu Cys Gly Val Lys Gly Asn Tyr Arg Thr Ile Gly Val
            100                 105                 110

Ser Ala Ala Ala Arg Tyr Asn Gln Cys Asn Thr Thr Arg Gly Asn Glu
        115                 120                 125

Val Thr Ser Val Ile Asn Arg Ala Lys Lys Ala Gly Lys Ala Val Gly
    130                 135                 140

Val Val Thr Thr Thr Arg Val Gln His Ala Ser Pro Ala Gly Ala Tyr
145                 150                 155                 160

Ala His Thr Val Asn Arg Asn Trp Tyr Ser Asp Ala Asp Leu Pro Ala
                165                 170                 175

Asp Ala Gln Lys Asn Gly Cys Gln Asp Ile Ala Ala Gln Leu Val Tyr
            180                 185                 190

Asn Met Asp Ile Asp Val Ile Leu Gly Gly Gly Arg Met Tyr Met Phe
        195                 200                 205

Pro Glu Gly Thr Pro Asp Pro Glu Tyr Pro Asp Asp Ala Ser Val Asn
    210                 215                 220

Gly Val Arg Lys Asp Lys Gln Asn Leu Val Gln Glu Trp Gln Ala Lys
225                 230                 235                 240

His Gln Gly Ala Gln Tyr Val Trp Asn Arg Thr Ala Leu Leu Gln Ala
                245                 250                 255

Ala Asp Asp Ser Ser Val Thr His Leu Met Gly Leu Phe Glu Pro Ala
            260                 265                 270

Asp Met Lys Tyr Asn Val Gln Gln Asp His Thr Lys Asp Pro Thr Leu
        275                 280                 285

Ala Glu Met Thr Glu Ala Ala Leu Gln Val Leu Ser Arg Asn Pro Arg
    290                 295                 300

Gly Phe Tyr Leu Phe Val Glu Gly Gly Arg Ile Asp His Gly His His
305                 310                 315                 320
```

Asp Phe Lys Ala Tyr Met Ala Leu Thr Glu Ala Ile Met Phe Asp Asn
325 330 335

Ala Ile Ala Lys Ala Asn Glu Leu Thr Ser Glu Leu Asp Thr Leu Ile
340 345 350

Leu Val Thr Ala Asp His Ser His Val Phe Ser Phe Gly Gly Tyr Thr
355 360 365

Leu Arg Gly Thr Ser Ile Phe Gly Leu Ala Pro Gly Lys Ala Leu Asp
370 375 380

Ser Lys Ser Tyr Thr Ser Ile Leu Tyr Gly Asn Gly Pro Gly Tyr Ala
385 390 395 400

Leu Gly Gly Gly Ser Arg Pro Asp Val Asn Gly Ser Thr Ser Glu Glu
405 410 415

Pro Ser Tyr Arg Gln Gln Ala Ala Val Pro Leu Ala Ser Glu Thr His
420 425 430

Gly Gly Glu Asp Val Ala Val Phe Ala Arg Gly Pro Gln Ala His Leu
435 440 445

Val His Gly Val Gln Glu Glu Thr Phe Val Ala His Ile Met Ala Phe
450 455 460

Ala Gly Cys Val Glu Pro Tyr Thr Asp Cys Asn Leu Pro Ala Pro Ala
465 470 475 480

Thr Ala Thr Ser Ile Pro Asp
485

<210> 6
<211> 487
<212> PRT
<213> Bovine/mutated

<400> 6

Leu Ile Pro Ala Glu Glu Glu Asn Pro Ala Phe Trp Asn Arg Gln Ala
1 5 10 15

Ala Gln Ala Leu Asp Val Ala Lys Lys Leu Gln Pro Ile Gln Thr Ala
20 25 30

Ala Lys Asn Val Ile Leu Phe Leu Gly Asp Gly Met Gly Val Pro Thr
35 40 45

Val Thr Ala Thr Arg Ile Leu Lys Gly Gln Met Asn Gly Lys Leu Gly
50 55 60

```
Pro Glu Thr Pro Leu Ala Met Asp Gln Phe Pro Tyr Val Ala Leu Ser
65                  70                  75                  80

Lys Thr Tyr Asn Val Asp Arg Gln Val Pro Asp Ser Ala Gly Thr Ala
                85                  90                  95

Thr Ala Tyr Leu Cys Gly Val Lys Gly Asn Tyr Arg Thr Ile Gly Val
            100                 105                 110

Ser Ala Ala Ala Arg Tyr Asn Gln Cys Asn Thr Thr Arg Gly Asn Glu
        115                 120                 125

Val Thr Ser Val Ile Asn Arg Ala Lys Lys Ala Gly Lys Ala Val Gly
    130                 135                 140

Val Val Thr Thr Thr Arg Val Gln His Ala Ser Pro Ala Gly Ala Tyr
145                 150                 155                 160

Ala His Thr Val Asn Arg Asn Trp Tyr Ser Asp Ala Asp Leu Pro Ala
                165                 170                 175

Asp Ala Gln Lys Asn Gly Cys Gln Asp Ile Ala Ala Gln Leu Val Tyr
            180                 185                 190

Asn Met Asp Ile Asp Val Ile Leu Gly Gly Gly Arg Met Tyr Met Phe
        195                 200                 205

Pro Glu Gly Thr Pro Asp Pro Glu Tyr Pro Asp Asp Ala Ser Val Asn
    210                 215                 220

Gly Val Arg Lys Asp Lys Gln Asn Leu Val Gln Glu Trp Gln Ala Lys
225                 230                 235                 240

His Gln Gly Ala Gln Tyr Val Trp Asn Arg Thr Ala Leu Leu Gln Ala
                245                 250                 255

Ala Asp Asp Ser Ser Val Thr His Leu Met Gly Leu Phe Glu Pro Ala
            260                 265                 270

Asp Met Lys Tyr Asn Val Gln Gln Asp His Thr Lys Asp Pro Thr Leu
        275                 280                 285

Ala Glu Met Thr Glu Ala Ala Leu Gln Val Leu Ser Arg Asn Pro Arg
    290                 295                 300

Gly Phe Tyr Leu Phe Val Glu Gly Gly Arg Ile Asp His Gly His Asn
305                 310                 315                 320

Asp Phe Lys Ala Tyr Met Ala Leu Thr Glu Ala Ile Met Phe Asp Asn
                325                 330                 335

Ala Ile Ala Lys Ala Asn Glu Leu Thr Ser Glu Leu Asp Thr Leu Ile
            340                 345                 350
```

```
Leu Val Thr Ala Asp His Ser His Val Phe Ser Phe Gly Gly Tyr Thr
        355                 360                 365

Leu Arg Gly Thr Ser Ile Phe Gly Leu Ala Pro Gly Lys Ala Leu Asp
    370                 375                 380

Ser Lys Ser Tyr Thr Ser Ile Leu Tyr Gly Asn Gly Pro Gly Tyr Ala
385                 390                 395                 400

Leu Gly Gly Gly Ser Arg Pro Asp Val Asn Gly Ser Thr Ser Glu Glu
                405                 410                 415

Pro Ser Tyr Arg Gln Gln Ala Ala Val Pro Leu Ala Ser Glu Thr His
            420                 425                 430

Gly Gly Glu Asp Val Ala Val Phe Ala Arg Gly Pro Gln Ala His Leu
        435                 440                 445

Val His Gly Val Gln Glu Glu Thr Phe Val Ala His Ile Met Ala Phe
    450                 455                 460

Ala Gly Cys Val Glu Pro Tyr Thr Asp Cys Asn Leu Pro Ala Pro Ala
465                 470                 475                 480

Thr Ala Thr Ser Ile Pro Asp
                485
```

<210> 7
<211> 487
<212> PRT
<213> Bovine/mutated

<400> 7

```
Leu Ile Pro Ala Glu Glu Glu Asn Pro Ala Phe Trp Asn Arg Gln Ala
1               5                   10                  15

Ala Gln Ala Leu Asp Val Ala Lys Lys Leu Gln Pro Ile Gln Thr Ala
            20                  25                  30

Ala Lys Asn Val Ile Leu Phe Leu Gly Asp Gly Met Gly Val Pro Thr
        35                  40                  45

Val Thr Ala Thr Arg Ile Leu Lys Gly Gln Met Asn Gly Lys Leu Gly
    50                  55                  60

Pro Glu Thr Pro Leu Ala Met Asp Gln Phe Pro Tyr Val Ala Leu Ser
65                  70                  75                  80

Lys Thr Tyr Asn Val Asp Arg Gln Val Pro Asp Ala Ala Gly Thr Ala
                85                  90                  95
```

Thr Ala Tyr Leu Cys Gly Val Lys Gly Asn Tyr Arg Thr Ile Gly Val
100 105 110

Ser Ala Ala Ala Arg Tyr Asn Gln Cys Asn Thr Thr Arg Gly Asn Glu
115 120 125

Val Thr Ser Val Ile Asn Arg Ala Lys Lys Ala Gly Lys Ala Val Gly
130 135 140

Val Val Thr Thr Thr Arg Val Gln His Ala Ser Pro Ala Gly Ala Tyr
145 150 155 160

Ala His Thr Val Asn Arg Asn Trp Tyr Ser Asp Ala Asp Leu Pro Ala
165 170 175

Asp Ala Gln Lys Asn Gly Cys Gln Asp Ile Ala Ala Gln Leu Val Tyr
180 185 190

Asn Met Asp Ile Asp Val Ile Leu Gly Gly Gly Arg Met Tyr Met Phe
195 200 205

Pro Glu Gly Thr Pro Asp Pro Glu Tyr Pro Asp Asp Ala Ser Val Asn
210 215 220

Gly Val Arg Lys Asp Lys Gln Asn Leu Val Gln Glu Trp Gln Ala Lys
225 230 235 240

His Gln Gly Ala Gln Tyr Val Trp Asn Arg Thr Ala Leu Leu Gln Ala
245 250 255

Ala Asp Asp Ser Ser Val Thr His Leu Met Gly Leu Phe Glu Pro Ala
260 265 270

Asp Met Lys Tyr Asn Val Gln Gln Asp His Thr Lys Asp Pro Thr Leu
275 280 285

Ala Glu Met Thr Glu Ala Ala Leu Gln Val Leu Ser Arg Asn Pro Arg
290 295 300

Gly Phe Tyr Leu Phe Val Glu Gly Gly Arg Ile Asp His Gly His Asn
305 310 315 320

Asp Phe Lys Ala Tyr Met Ala Leu Thr Glu Ala Ile Met Phe Asp Asn
325 330 335

Ala Ile Ala Lys Ala Asn Glu Leu Thr Ser Glu Leu Asp Thr Leu Ile
340 345 350

Leu Val Thr Ala Asp His Ser His Val Phe Ser Phe Gly Gly Tyr Thr
355 360 365

Leu Arg Gly Thr Ser Ile Phe Gly Leu Ala Pro Gly Lys Ala Leu Asp
370 375 380

```
Ser Lys Ser Tyr Thr Ser Ile Leu Tyr Gly Asn Gly Pro Gly Tyr Ala
385                 390                 395                 400

Leu Gly Gly Gly Ser Arg Pro Asp Val Asn Gly Ser Thr Ser Glu Glu
                405                 410                 415

Pro Ser Tyr Arg Gln Gln Ala Ala Val Pro Leu Ala Ser Glu Thr His
            420                 425                 430

Gly Gly Glu Asp Val Ala Val Phe Ala Arg Gly Pro Gln Ala His Leu
        435                 440                 445

Val His Gly Val Gln Glu Glu Thr Phe Val Ala His Ile Met Ala Phe
    450                 455                 460

Ala Gly Cys Val Glu Pro Tyr Thr Asp Cys Asn Leu Pro Ala Pro Ala
465                 470                 475                 480

Thr Ala Thr Ser Ile Pro Asp
                485
```

<210> 8
<211> 1476
<212> DNA
<213> Nucleic acid

<400> 8

```
gaattcttga ttccagctga agaagaaaat ccagcttttt ggaatagaca agctgctcaa      60
gctttggatg ttgctaagaa gttgcaacca attcaaactg ctgctaagaa tgttattttg     120
tttttgggtg atggtatggg tgttccaact gttactgcta ctagaatttt gaagggtcaa     180
atgaatggta agttgggtcc agaaactcca ttggctatgg atcaatttcc atacgttgct     240
ttgtctaaga cttacaatgt tgatagacaa gttccagatg ctgctggtac tgctactgct     300
tacttgtgtg gtgttaaggg taattacaga actattggtg tttctgctgc tgctagatac     360
aatcaatgta atactactag aggtaatgaa gttacttctg ttattaatag agctaagaag     420
gctggtaagg ctgttggtgt tgttactact actagagttc aacatgcttc tccagctggt     480
gcttacgctc atactgttaa tagaaattgg tactctgatg ctgatttgcc agctgatgct     540
caaaagaatg gttgtcaaga tattgctgct caattggttt acaatatgga tattgatgtt     600
attttgggtg gtggtagaat gtacatgttt ccagaaggta ctccagatcc agaataccca     660
gatgatgctt ctgttaatgg tgttagaaag gataagcaaa atttggttca agaatggcaa     720
```

```
gctaagcatc aaggtgctca atatgtttgg aatagaactg ctttgttgca agctgctgat    780
gattctagtg ttactcattt gatgggtttg tttgaaccag ctgatatgaa gtataatgtt    840
caacaagatc atactaagga tccaactttg gctgaaatga ctgaagctgc tttgcaagtt    900
ttgtctagaa atccaagagg tttttacttg tttgttgaag gtggtagaat tgatcatggt    960
catcatgatg gtaaggctta tatggctttg actgaagcta ttatgtttga taatgctatt   1020
gctaaggcta atgaattgac ttctgaattg gatactttga ttttggttac tgctgatcat   1080
agtcatgttt tttcttttgg tggttacact ttgagaggta cttctatttt tggtttggct   1140
ccaggtaagg ctttggatag taagtcttac acttctattt tgtatggtaa tggtccaggt   1200
tatgctttgg gtggtggttc tagaccagat gttaatggta gtactagtga agaaccatct   1260
tacagacaac aagctgctgt tccattggct agtgaaactc atggtggtga agatgttgct   1320
gtttttgcta gaggtccaca agctcatttg gttcatggtg ttcaagaaga aacttttgtt   1380
gctcatatta tggcttttgc tggttgtgtt gaaccataca ctgattgtaa tttgccagct   1440
ccagctactg ctactagtat tccagattaa ggtacc                             1476
```

<210> 9
<211> 1476
<212> DNA
<213> Nucleic acid

<400> 9

```
gaattcttga ttccagctga agaagaaaat ccagcttttt ggaatagaca agctgctcaa     60
gctttggatg ttgctaagaa gttgcaacca attcaaactg ctgctaagaa tgttattttg    120
tttttgggtg atggtatggg tgttccaact gttactgcta ctagaatttt gaagggtcaa    180
atgaatggta agttgggtcc agaaactcca ttggctatgg atcaatttcc atacgttgct    240
ttgtctaaga cttacaatgt tgatagacaa gttccagatt ctgctggtac tgctactgct    300
tacttgtgtg gtgttaaggg taattacaga actattggtg tttctgctgc tgctagatac    360
aatcaatgta atactactag aggtaatgaa gttacttctg ttattaatag agctaagaag    420
gctggtaagg ctgttggtgt tgttactact actagagttc aacatgcttc tccagctggt    480
gcttacgctc atactgttaa tagaaattgg tactctgatg ctgatttgcc agctgatgct    540
caaaagaatg gttgtcaaga tattgctgct caattggttt acaatatgga tattgatgtt    600
```

```
attttgggtg gtggtagaat gtacatgttt ccagaaggta ctccagatcc agaataccca    660
gatgatgctt ctgttaatgg tgttagaaag gataagcaaa atttggttca agaatggcaa    720
gctaagcatc aaggtgctca atatgtttgg aatagaactg ctttgttgca agctgctgat    780
gattctagtg ttactcattt gatgggtttg tttgaaccag ctgatatgaa gtataatgtt    840
caacaagatc atactaagga tccaactttg gctgaaatga ctgaagctgc tttgcaagtt    900
ttgtctagaa atccaagagg tttttacttg tttgttgaag gtggtagaat tgatcatggt    960
catcatgatt ttaaggctta tatggctttg actgaagcta ttatgtttga taatgctatt   1020
gctaaggcta atgaattgac ttctgaattg gatactttga ttttggttac tgctgatcat   1080
agtcatgttt tttcttttgg tggttacact ttgagaggta cttctatttt tggtttggct   1140
ccaggtaagg ctttggatag taagtcttac acttctattt tgtatggtaa tggtccaggt   1200
tatgctttgg gtggtggttc tagaccagat gttaatggta gtactagtga agaaccatct   1260
tacagacaac aagctgctgt tccattggct agtgaaactc atggtggtga agatgttgct   1320
gtttttgcta gaggtccaca agctcatttg gttcatggtg ttcaagaaga aacttttgtt   1380
gctcatatta tggcttttgc tggttgtgtt gaaccataca ctgattgtaa tttgccagct   1440
ccagctactg ctactagtat tccagattaa ggtacc                             1476
```

<210> 10
<211> 1476
<212> DNA
<213> Nucleic acid

<400> 10

```
gaattcttga ttccagctga agaagaaaat ccagcttttt ggaatagaca agctgctcaa     60
gctttggatg ttgctaagaa gttgcaacca attcaaactg ctgctaagaa tgttattttg    120
tttttgggtg atggtatggg tgttccaact gttactgcta ctagaatttt gaagggtcaa    180
atgaatggta agttgggtcc agaaactcca ttggctatgg atcaatttcc atacgttgct    240
ttgtctaaga cttacaatgt tgatagacaa gttccagatt ctgctggtac tgctactgct    300
tacttgtgtg gtgttaaggg taattacaga actattggtg tttctgctgc tgctagatac    360
aatcaatgta atactactag aggtaatgaa gttacttctg ttattaatag agctaagaag    420
gctggtaagg ctgttggtgt tgttactact actagagttc aacatgcttc tccagctggt    480
```

gcttacgctc atactgttaa tagaaattgg tactctgatg ctgatttgcc agctgatgct 540

caaaagaatg gttgtcaaga tattgctgct caattggttt acaatatgga tattgatgtt 600

attttgggtg gtggtagaat gtacatgttt ccagaaggta ctccagatcc agaataccca 660

gatgatgctt ctgttaatgg tgttagaaag gataagcaaa atttggttca agaatggcaa 720

gctaagcatc aaggtgctca atatgtttgg aatagaactg ctttgttgca agctgctgat 780

gattctagtg ttactcattt gatgggtttg tttgaaccag ctgatatgaa gtataatgtt 840

caacaagatc atactaagga tccaactttg gctgaaatga ctgaagctgc tttgcaagtt 900

ttgtctagaa atccaagagg tttttacttg tttgttgaag gtggtagaat tgatcatggt 960

cataatgatt ttaaggctta tatggctttg actgaagcta ttatgtttga taatgctatt 1020

gctaaggcta atgaattgac ttctgaattg gatactttga ttttggttac tgctgatcat 1080

agtcatgttt tttcttttgg tggttacact ttgagaggta cttctatttt tggtttggct 1140

ccaggtaagg ctttggatag taagtcttac acttctattt tgtatggtaa tggtccaggt 1200

tatgctttgg gtggtggttc tagaccagat gttaatggta gtactagtga agaaccatct 1260

tacagacaac aagctgctgt tccattggct agtgaaactc atggtggtga agatgttgct 1320

gtttttgcta gaggtccaca agctcatttg gttcatggtg ttcaagaaga aacttttgtt 1380

gctcatatta tggcttttgc tggttgtgtt gaaccataca ctgattgtaa tttgccagct 1440

ccagctactg ctactagtat tccagattaa ggtacc 1476

<210> 11
<211> 1476
<212> DNA
<213> Nucleic acid

<400> 11

gaattcttga ttccagctga agaagaaaat ccagcttttt ggaatagaca agctgctcaa 60

gctttggatg ttgctaagaa gttgcaacca attcaaactg ctgctaagaa tgttattttg 120

tttttgggtg atggtatggg tgttccaact gttactgcta ctagaatttt gaagggtcaa 180

atgaatggta agttgggtcc agaaactcca ttggctatgg atcaatttcc atacgttgct 240

ttgtctaaga cttacaatgt tgatagacaa gttccagatg ctgctggtac tgctactgct 300

tacttgtgtg gtgttaaggg taattacaga actattggtg tttctgctgc tgctagatac 360

```
aatcaatgta atactactag aggtaatgaa gttacttctg ttattaatag agctaagaag    420
gctggtaagg ctgttggtgt tgttactact actagagttc aacatgcttc tccagctggt    480
gcttacgctc atactgttaa tagaaattgg tactctgatg ctgatttgcc agctgatgct    540
caaaagaatg gttgtcaaga tattgctgct caattggttt acaatatgga tattgatgtt    600
attttgggtg gtggtagaat gtacatgttt ccagaaggta ctccagatcc agaataccca    660
gatgatgctt ctgttaatgg tgttagaaag gataagcaaa atttggttca agaatggcaa    720
gctaagcatc aaggtgctca atatgtttgg aatagaactg ctttgttgca agctgctgat    780
gattctagtg ttactcattt gatgggtttg tttgaaccag ctgatatgaa gtataatgtt    840
caacaagatc atactaagga tccaactttg gctgaaatga ctgaagctgc tttgcaagtt    900
ttgtctagaa atccaagagg tttttacttg tttgttgaag gtggtagaat tgatcatggt    960
cataatgatt ttaaggctta tatggctttg actgaagcta ttatgtttga taatgctatt   1020
gctaaggcta atgaattgac ttctgaattg gatactttga ttttggttac tgctgatcat   1080
agtcatgttt tttcttttgg tggttacact ttgagaggta cttctatttt tggtttggct   1140
ccaggtaagg ctttggatag taagtcttac acttctattt tgtatggtaa tggtccaggt   1200
tatgctttgg gtggtggttc tagaccagat gttaatggta gtactagtga agaaccatct   1260
tacagacaac aagctgctgt tccattggct agtgaaactc atggtggtga agatgttgct   1320
gtttttgcta gaggtccaca agctcatttg gttcatggtg ttcaagaaga aacttttgtt   1380
gctcatatta tggcttttgc tggttgtgtt gaaccataca ctgattgtaa tttgccagct   1440
ccagctactg ctactagtat tccagattaa ggtacc                              1476
```

<210> 12
<211> 46
<212> DNA
<213> Primer 5'-hAP

<400> 12
gcgcgaattc ttgattccag ctgaagaaga aaatccagct ttttgg 46

<210> 13
<211> 47
<212> DNA
<213> Primer 3'-hAP

<400> 13
gcgcggtacc ttaatctgga atactagtag cagtagctgg agctggc 47

<210> 14
<211> 26
<212> DNA
<213> Primer 5'-S92A

<400> 14
ccagatgctg ctggtactgc tactgc 26

<210> 15
<211> 34
<212> DNA
<213> Primer 3'-S92A

<400> 15
gcagtagcag taccagcagc atctggaact tgtc 34

<210> 16
<211> 35
<212> DNA
<213> Primer 5'-G322H

<400> 16
gatcatggtc atcatgattt taaggcttat atggc 35

<210> 17
<211> 35
<212> DNA
<213> Primer 3'-G322H

<400> 17
gccatataag ccttaaaatc atgatgacca tgatc 35

<210> 18
<211> 35
<212> DNA
<213> Primer 5'-H320N/G322H

<400> 18
gatcatggtc ataatgattt taaggcttat atggc 35

<210> 19
<211> 35
<212> DNA
<213> Primer 5'H320N/G322F

<400> 19
gccatataag ccttaaaatc attatgacca tgatc 35

<210> 20
<211> 31
<212> DNA
<213> Primer 5'-Expr

<400> 20
gcgcgcctag gagatctaac atccaaagac g 31

<210> 21
<211> 29
<212> DNA
<213> Primer 3'-Expr

<400> 21
cgcgcgctag cggatccgca caaacgaag 29